# EUROPEAN PATENT APPLICATION

(11) **EP 4 733 298 A1**
(43) Date of publication of application: **29.04.2026**
(21) Application number: 24207960.6
(22) Date of filing: 22.10.2024
(51) Int. Cl.: C07D 263/56, C07D 413/12, A61P 9/00, A61P 25/00, A61P 37/00, A61K 31/423

(54) **NOVEL TRPM4 ANTAGONISTS AND MEDICAL USE THEREOF**

(71) Applicant: Universitätsklinikum Hamburg-Eppendorf, 20246 Hamburg (DE)
(72) Inventor: Friese, Manuel Alexander, 20246 Hamburg (DE); Binkle, Lars, 20246 Hamburg (DE)
(74) Representative: Eisenführ Speiser

(57) **Abstract**

The invention relates to a compound which is effective in inhibiting the function of the TRPM4 ion channel and the use of such compound in treating or preventing a TRPM4-associated disorder in a subject such as neurodegenerative diseases like stroke, Alzheimer's disease, Parkinson's disease, amyotrophic lateral sclerosis, and neurodegenerative disease under inflammatory conditions, in particular multiple sclerosis, or genodermatosis, cardiac diseases, and immune-mediated diseases. The invention also provides a pharmaceutical composition comprising the TRPM4 inhibitory compound.

## Description

The invention relates to a compound which is effective in inhibiting the function of the TRPM4 ion channel and the use of such compound in treating or preventing a TRPM4-associated disorder in a subject such as neurodegenerative diseases like stroke, Alzheimer's disease, Parkinson's disease, amyotrophic lateral sclerosis, and neurodegenerative disease under inflammatory conditions, in particular multiple sclerosis, or genodermatosis, cardiac diseases, and immune-mediated diseases. The invention also provides a pharmaceutical composition comprising the TRPM4 inhibitory compound.

### BACKGROUND OF THE INVENTION

Neurons are characterized by their remarkable electrical excitability and elaborated networks distinguishing them from other cell types. This excitability is facilitated by an electrochemical gradient, primarily established through the activity of the potassium-sodium pump, allowing subsequent ion movement between the cytosol and the extracellular space via ion channels in the plasma membrane ¹. However, this distinctive feature can become a liability when neuronal excitation surpasses physiological limits in central nervous system (CNS) disorders. Glutamate, the primary excitatory neurotransmitter in the mammalian nervous system, is intricately linked to tightly regulated intracellular calcium levels that are essential for numerous cellular processes ^{2,3}. Specifically, synaptic release of glutamate that activates N-methyl-D-aspartate receptors (NMDAR) is crucial for calcium influx, initiating a cascade of calcium-dependent processes, that are for instance involved in learning and memory and are required for neuronal survival ^{4,5}. In contrast, pathologically excessive glutamate release can overburden this system, leading to calcium-dependent neuronal damage and cell death known as excitotoxicity ⁶⁻⁹.

Several mechanisms contribute to elevated extracellular glutamate levels. Although astrocytes efficiently remove excess glutamate from the extracellular space, they can also release glutamate to modulate neuronal activity ¹⁰. Excessive neuronal activity may cause glutamate spillover from synaptic sites, activating extra-synaptic NMDARs ⁸. During neuroinflammation, activated microglia ¹¹⁻¹³ and infiltrating T cells ¹⁴ serve as additional sources of glutamate. Consequently, excitotoxicity is a significant contributor to neuronal cell death in various acute and chronic neurodegenerative disorders such as stroke, Alzheimer's disease (AD), Parkinson's disease (PD), amyotrophic lateral sclerosis (ALS), and multiple sclerosis (MS) ¹⁵⁻¹⁷.

Pharmaceutical targeting of NMDARs or glutamate metabolism has yielded unsatisfying outcomes. Interfering with neurotransmitter levels or synaptic transmission poses the risk of severe side effects, complicating therapy development ¹⁸⁻²¹. An alternative approach involves targeting cytotoxic events downstream of the NMDAR, with the goal of attenuating adverse effects while mitigating the deleterious effects of neuronal excitotoxicity ²²⁻²⁶. In this regard, the transient receptor potential melastatin 4 (TRPM4) channel (SEQ ID NO: 1), which functions as a calcium-activated non-selective cation channel capable of amplifying toxic NMDAR signaling ²⁴, has emerged as a promising therapeutic target.

TRPM4 belongs to the evolutionarily conserved family of transient receptor potential (TRP) channels. This family consists of 28 cation permeable channels with six putative transmembrane domains that are grouped into six subfamilies ²⁷. The melastatin-related TRP (TRPM) subfamily consists of eight members that can be further divided into four groups ²⁸. TRPM4 (SEQ ID NO: 1) and the closest homolog TRPM5 (SEQ ID NO: 4) distinguish themselves from other members of the TRPM family by the capacity to only conduct monovalent cations. TRPM5 is primarily expressed in pancreatic beta-cells, intestinal and taste cells and is particularly important for taste signaling ²⁹. In contrast, TRPM4 is widely expressed throughout the body and is activated by intracellular calcium binding, coordinated by conserved amino acids of the S2 and S3 transmembrane helices ³⁰. Channel gating is also sensitive to membrane potential ³¹, phosphoinositide lipids in the plasma membrane ^{31,32} and the local concentration of cytoplasmic ATP ³³. Sulfonylurea receptor 1 (SUR1) has been proposed as auxiliary subunit of the TRPM4 channel and SUR1-TRPM4 was found to form a complex with aquaporin-4 (AQP4), regulating water influx at the astrocytic end feet ^{34,35}. Glibenclamide, a US Food and Drug Administration (FDA)-approved oral antidiabetic drug (also known as glyburide), has been shown to block SUR1-associated channels including TRPM4 ³⁶⁻³⁸.

TRPM4 also binds to the NMDAR and regulates its subcellular localization. Pharmacological dissociation of the neuronal TRPM4-NMDAR complex or glibenclamide treatment improve the outcome of ischemic stroke-induced brain damage in mice ^{24,38}. Moreover, glibenclamide treatment protects from neurodegeneration in a mouse model of neuroinflammation ³⁹. Genetic ablation or antibody blocking of TRPM4 was shown to be beneficial in mouse models of MS and ischemic stroke without detectable impairments due to loss of TRPM4 function ^{35,39-41}. Beyond neurological disorders ^{39,42,43}, TRPM4 has been associated with a variety of conditions, including genodermatosis ⁴⁴, cardiac ⁴⁵⁻⁵⁰, and immune-mediated ⁵¹⁻⁵⁴ diseases. In all of these cases, the primary underlying cause is an increase in TRPM4 function by gain-of-function mutations, *de novo* expression or up-regulation of TRPM4 expression. Consequently, antagonizing TRPM4 function is a promising strategy for the treatment of various diseases, in particular neurodegeneration.

Halting neurodegeneration in CNS disorders is an unmet medical need, particularly in ageing societies ^{15,71-74}. It is now increasingly recognized that many neurodegenerative disorders share common molecular pathways leading to neuronal cell death. One predominant pathway is the excessive accumulation of calcium in neurons, which can be triggered by excitotoxicity ^{75,76}. TRPM4 emerges as a promising target to counteract neuronal demise due to its interaction with NMDAR and downstream signaling pathways.

A number of inhibitors of TRPM4 have been described in the art. For example, nucleotides such as ATP, ADP, AMP, AMP-PNP, and adenosine have been described to inhibit the TRPM4 ion channel quickly and reversibly. Similarly, polyamines like spermine have also been found to block TRPM4 currents (Nilius et al. (2004), Eur J Physiol. 448:70-75).

Other known TRPM4 antagonists are anthranilic acid orthologs such as flufenamic acid (FFA)⁵⁹ and CBA⁵⁷, and meclofenamate⁴⁵. However, meclofenamate is known to be associated with adverse effects, such as behavioral alterations or other adverse effects in treated animals.⁴⁵

Other compounds that have been described to inhibit the TRPM4 ion channel are those referred to in WO 2006/034048 and WO 03/079987. These compounds include antagonists to sulfonylurea receptor-1 (SUR1), such as 9-phenanthrol, glibenclamide, tolbutamide, repaglinide, nateglinide, meglitinide, midaglizole, LY397364, LY389382, glyclazide, glimepiride, estrogen, and estrogen-related compounds (such as estradiol, estrone, estriol, genistein, non-steroidal estrogen, phytoestrogen, zearalenone, and the like).

However, until today there are no TRPM4 drugs approved for human use except glibenclamide and meclofenamate, which suffer from low affinity and lack of specificity ^{45,55,56}. Only recently, advancements have led to the development of anthranilic acid derivatives, including 4-chloro-2-[[2-(2-chlorophenoxy)acetyl]amino]benzoic acid (CBA), with better antagonistic affinity ⁵⁷. However, none of these anthranilic acid derivatives have been successfully applied in preclini-cal models.

Accordingly, there is a need for improved TRPM4 drugs.

The present inventors surprisingly found that compounds disclosed herein effectively inhibit the function of the TRPM4 ion channel and thus are potent and highly specific TRPM4 antagonists. The present inventors surprisingly found that compounds disclosed herein are able to reduce neuronal excitotoxicity.

Additionally, the present inventors identified a specific binding site for these compounds on TRPM4, substantiated by mutational analysis and electrophysiological studies.

The present invention therefore provides a new and widely applicable therapeutic strategy for treating or preventing TRPM4-associated disorders in a subject such as neurodegenerative diseases like stroke, Alzheimer's disease, Parkinson's disease, amyotrophic lateral sclerosis, and neurodegenerative disease under inflammatory conditions, in particular multiple sclerosis, or genodermatosis, cardiac diseases, and immune-mediated diseases.

### DESCRIPTION OF THE INVENTION

The present invention is amongst other things based on the insight that blocking the TRPM4 ion channel confers resistance of axons and neurons towards hostile challenges which results in neuro-axonal preservation and less clinical disability and neurodegeneration. Therefore, the present invention specifically contemplates to use antagonists and/or inhibitors of the TRPM4 ion channel for treating or preventing a TRPM4-associated disorder such as neurodegenerative diseases like stroke, Alzheimer's disease, Parkinson's disease, amyotrophic lateral sclerosis, and neurodegenerative disease under inflammatory conditions, in particular multiple sclerosis, or genodermatosis, cardiac diseases, and immune-mediated diseases in a subject.

Thus, in a first aspect the invention relates to a compound as defined herein effective in inhibiting the function of a TRPM4 ion channel for use in a method of treating or preventing a TRPM4-associated disorder in a subject.

As used herein, the "function of a TRPM4 ion channel" means the capability of the protein to regulate the influx of ions into the cell, in particular the influx of cations such as Na⁺. Accordingly, a "functional" TRPM4 ion channel refers to a channel protein that effectively regulates the influx of ions, such as Na⁺, into the cell The present invention is hence useful for treating or ameliorating the effects of TRPM4-associated disorder, in particular neurodegenerative diseases, which are associated with TRPM4-mediated cytotoxicity.

The compounds of the present invention effectively antagonize TRPM4 function, reduce neuronal excitotoxicity and/or prevent damage and/or loss of neurons in the nervous system (NS) of a subject, preferably in the central nervous system (CNS) of a subject. As used herein, the NS is to be understood as being composed of the CNS and the peripheral nervous system. Further, as used herein, the CNS is to be understood as containing the brain and the spinal cord. In a preferred embodiment, the compounds of the invention are administered for preventing damage and/or loss of neurons in the brain. In a further embodiment, progressive damage and/or loss of neurons may be halted by administration of the compounds of the invention. Halting the damage and/or the loss of neurons means that the pathological processes of the neurodegenerative disease which finally result in damage and/or loss of neurons are stopped or at least reduced.

The TRPM4-associated disorder to be treated or prevented according to the invention may be any known TRPM4-associated disorder, preferably one that is associated with glutamate excitotoxicity. It has been found that TRPM4 signalling contributes to glutamate excitotoxicity which has devastating effects in a large number of neurodegenerative diseases. Therefore, the compounds, methods and uses of the present invention will be particularly useful in the treatment of TRPM4-associated disorders, in particular neurodegenerative diseases, which have been associated with glutamate excitotoxicity.

Thus, according to a preferred embodiment, the TRPM4-associated disorder to be treated or prevented according to the invention is known to be associated with glutamate excitotoxicity.

As used herein, "glutamate excitotoxicity" refers to a process that results in damaging or killing neuronal and/or axonal cells by excessive stimulation of these cells by glutamate and other substances that are capable of activating the glutamate receptor. Thus, glutamate excitotoxicity occurs as a result from overactivation of glutamate receptors. For example, the neurodegenerative disease known to be associated with glutamate excitotoxicity may be selected from the group Multiple Sclerosis (MS), such as relapsing remitting MS or secondary progressive MS, Parkinson's disease, Alzheimer's disease, and amyotrophic lateral sclerosis. In a particularly preferred embodiment, the neurodegenerative disease is MS.

In embodiments, the TRPM4-associated disorder may be selected from neurodegenerative diseases, genodermatosis, cardiac diseases, and immune-mediated diseases, preferably the TRPM4-associated disorder is a neurodegenerative disease.

The neurodegenerative disease to be treated or prevented according to the invention may be any known neurodegenerative disease. As used herein a neurodegenerative disease is a non-traumatic, disease which is associated with the progressive loss of functional neurons in the NS, preferably the CNS. The neurodegenerative disease to be treated according to the invention may be caused by a genetic predisposition. Conditions of the NS elicited traumatic events and/or physical shock, such as traumatic brain injury, cerebral ischemia, hypoxia and edema are not understood as neurodegenerative diseases in the sense of the present invention. The neurodegenerative disease is preferably one that is associated with inflammation, in particular multiple sclerosis. In other embodiments, the neurodegenerative disease selected from stroke, Alzheimer's disease, Parkinson's disease, amyotrophic lateral sclerosis.

### Inhibitory compounds

The compounds which are contemplated herein for treating and/or preventing a neurodegenerative disease are effective in inhibiting the function of the TRPM4 ion channel (SEQ ID NO: 1). This means that the compounds effectively reduce the extent of membrane current that occurs due to the influx of cations upon opening of the channel. In a preferred embodiment, the compound decreases the TRPM4 ion channel (SEQ ID NO: 1) activity by at least 10%, 20%, 30%, 40%, 50%, 60%, 70%, 80%, 90%, or 95% compared to the activity in the absence of the compound. In a particularly preferred embodiment, the inhibitory compound blocks the TRPM4 ion channel (SEQ ID NO: 1), i.e. the channel is completely deactivated so that there is no detectable influx of cations, such as Na⁺. The compounds preferably act by interacting with or binding to the TRPM4 protein (SEQ ID NO: 1), thereby preventing the sterical changes in the channel protein that occur upon opening of the channel, e.g. in response to an increase in the intracellular Ca²⁺ concentration. Compounds that actively bind to TRPM4 (SEQ ID NO: 1) may bind to the extracellular, the intracellular or the transmembrane part of the TRPM4 ion channel protein.

Preferably, the inhibitory compound is specific for the TRPM4 ion channel (SEQ ID NO: 1), i.e. the compound inhibits the expression and/or activity of the TRPM4 ion channel, while it does essentially not inhibit the expression and/or activity of other proteins or enzymes, e.g. other ion channels. In other words, the compounds according to the present invention essentially do not provide for undesired off-target effects. As a result of the TRPM4 specificity, the compounds according to the invention elicit no or only tolerable side effects when administered to a subject. This renders the compounds disclosed herein safe for application in humans.

The compounds contemplated by the invention affect the function of a TRPM4 ion channel protein (SEQ ID NO: 1). In a preferred embodiment, the inhibitory compounds interfere with the human TRPM4 ion channel protein. In the context of the present invention, the terms "TRPM4 ion channel" or "TRPM4" refer to the calcium-activated transient receptor potential melastatin 4 cation channel (SEQ ID NO: 1). TRPM4 belongs to the family of transient receptor potential cation channels, and more specifically to the subfamily M of this family of cation channels.

The human TRPM4 protein occurs in three different isoforms that are depicted in SEQ ID NO:1 (NP_060106), SEQ ID NO:2 (NP_001182156) and SEQ ID NO: 3 (NP_001308212.1). Preferably, the compounds contemplated by the invention inhibit the function of an TRPM4 ion channel having an amino acid sequence as depicted in SEQ ID NO: 1 (NP_060106) or an amino acid sequence having at least 90% sequence identity to the sequence of SEQ ID NO:1 (NP_060106). In a further preferred embodiment, the compounds contemplated by the invention inhibit the function of an TRPM4 ion channel having an amino acid sequence as depicted in SEQ ID NO:2 (NP_001182156) or an amino acid sequence having at least 90% sequence identity to the sequence of SEQ ID NO:2 (NP_001182156). In a further preferred embodiment, the compounds contemplated by the invention inhibit the function of an TRPM4 ion channel having an amino acid sequence as depicted in SEQ ID NO:3 (NP _001308212.1) or an amino acid sequence having at least 90% sequence identity to the sequence of SEQ ID NO:2 (NP_001308212.1). Further isoforms of the human TRPM4 protein which retain the ion channel activity may also be inhibited by the compounds of the present invention.

In preferred embodiments, the compounds according to the invention binds to TRPM4 at least via amino acids L907 and S924 of SEQ ID NO: 1.

The subject to be treated with the inhibitory compound will normally be a mammal, and preferably is a human. Generally, the subject can be of any age. The subject preferably suffers from a neurodegenerative disease, more preferably from a progressive neurodegenerative disease. The subject may be in any stage of the disease, i.e. the disease may be in an early stage wherein the subject shows only the first pathological signs that are normally associated with said disease, or the subject may be in a late stage of the disease. The subject to be treated may have a genetic or other predisposition for developing a neurodegenerative disease in the future.

The TRPM4-inhibitory compound of the invention can be derived from different groups of molecules selected from benzoxazole according to Formula (I), anthranilic acid according to Formula (II), 5-(5-Isoxazolyl)-2-thiophenesulfonamide according to Formula (III), benzpyrimidine according to Formula (IV), and/or thiadiazole according to Formula (V) as described hereinbelow.

The present inventors have surprisingly found that compounds as defined in the claims advantageously provide for IC50 values in the nanomolar range, as well as low toxicity and minimal off-target effects at the same time. This renders the compounds according to the invention particularly suitable for treating or preventing TRPM4-associated disorders in a subject, in particular neurodegenerative diseases.

The compounds according to the invention have a common compound binding site on TRPM4 (SEQ ID NO: 1), which is suggested by data from electrophysiological analysis of binding site mutants.

General and preferred aspects of the compounds of the present invention are disclosed below and in the appended claims.

R-groups discussed herein (also referred to substituents) are designated according to common nomenclature in organic chemistry. The skilled person understands that, if not otherwise stated, hydrogen atoms as substituents on carbon atoms are implicitly shown in structures. Also substituents and R groups are understood to have common valency to form covalent bonds with the carbon or hetero atom they are attached to. For example, if it is stated that an R group at is selected from H, F, Cl, Br, I, substituted or unsubstituted C₁ to C₁₀ alkyl, the skilled person understands that said R groups are radical substituents. In an alternative way, a radical substituent is indicated by a "wavey" bond 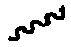 such as in the example of 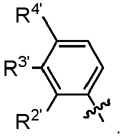 Also, by way of example, substituents designated as "NH₂" or "OMe" are understood to refer to amino groups or methoxy groups, respectively.

The term "C₁ to C₁₀ alkyl" as used herein refers to a straight or branched saturated hydrocarbon chain containing from 1 to 10 carbon atoms. Examples include methyl, ethyl, n-propyl, isopropyl (iPr), n-butyl (nBu), s-butyl, t-butyl (tBu), n-hexyl, n-octyl and n-decyl.

The term "C₁ to C₄ alkyl" as used herein refers to a straight or branched saturated hydrocarbon chain containing from 1 to 4 carbon atoms. Examples include methyl, ethyl, n-propyl, isopropyl (iPr), n-butyl (nBu),

The term "substituted" as used herein in context of an alkyl group means that an hydrogen atom of the alkyl group is replaced by a substituent, e.g. a halogen radical substituents, in particular fluoro, chloro, bromo or iodo. An example of a substituted methyl group is a trifluoromethyl group in which three hydrogen atoms are replaced by flouro radical substituents.

The term "halosubstituted" as used herein in context of an alkyl group means that an hydrogen atom of the alkyl group is replaced by a halogen radical substituents. As an example, the skilled person understands that a trifluoromethyl group is a halosubstituted methyl group.

The term "heteroaryl" as used herein refers to any hydrocarbon group that includes one or more aromatic ring that includes one or more heteroatom (e.g. N, O or S) as part of said ring. Examples of heteroaryl groups are pyridine, furan, thiophene.

The term "annulated" as used herein in context of aryl groups refers to condensed aromatic ring systems. An example of an annulated C₁₀ aryl group, which could also be referred to as a "10-membered annulated aryl group", is a naphtyl group. In contrast, the term "non-annulated" as used herein in context of aryl groups refers to aromatic ring systems which are not condensed. An example of a non-annulated C₆ aryl group, which could also be referred to as a "6-membered non-annulated aryl group", is a phenyl group.

The compounds as disclosed herein may be in the form of a pharmaceutically acceptable salt, solvate, tautomer or ester thereof. Salts of the compounds disclosed herein are suitably pharmaceutically or veterinary acceptable salts. Depending on the nature of the individual compounds, these may be basic addition salts such as sodium, potassium, calcium, aluminium, zinc, magnesium and other metal salts as well as choline, diethanolamine, ethanolamine, ethyl diamine, megulmine and other well-known basic addition salts as summarised in Paulekuhn et al., (2007) J. Med. Chem. 50: 6665-6672 and/or known to those skilled in the art. Alternatively, when a compound as disclosed herein contains an amino group, this may be quaternised to form a salt with a counter ion such as halide, hydroxide, sulfate, nitrate, phosphate, formate, acetate, trifluoroacetate, fumarate, citrate, tartrate, oxalate, succinate, mandelate, methane sulfonate and p-toluene sulfonate.

### Benzoxazole according to Formula (I)

In one embodiment, the compound is a benzoxazole according to 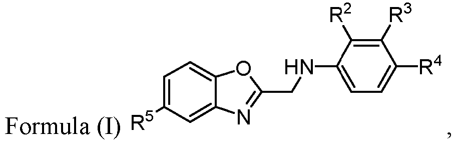 wherein
R2 is selected from H, F, Cl, Br, I, substituted or unsubstituted C₁ to C₁₀ alkyl, in particular CF₃, substituted or unsubstituted aryl or heteroaryl groups, amine groups NR⁶R⁷ with R6 and R7 independently selected from H, and C₁ to C₄ alkyl, in particular, Me, Et, Pr, iPr, hydroxy or alkoxy groups OR⁸ with R8 being selected from H, and C₁ to C₄ alkyl, in particular, Me, Et, Pr, iPr, Bu and 5 to 10 membered non-annulated or annulated aryl or heteroaryl,
   preferably R2 is
   phenyl according to 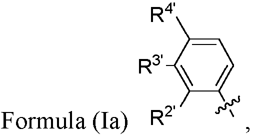 wherein
      R2', R3' and R4' are independently selected from H, F, Cl, Br, I, substituted or unsubstituted C₁ to C₁₀ alkyl, in particular CF₃, amine groups NR^{6'}R^{7'} with R6' and R7' independently selected from H, and C₁ to C₄ alkyl, in particular, Me, Et, Pr, iPr, Bu and hydroxy or alkoxy groups OR^{8'} with R8' being selected from H, and C₁ to C₄ alkyl, in particular, Me, Et, Pr, iPr, Bu;
      preferably R2' is selected from H, F and NH₂, R3' is selected from H, NH₂, NHMe and NHiPr, and R4' is selected from H and NHMe;
         or
   heteroaryl according to 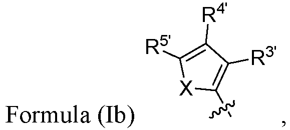 wherein
      X is selected from N, O and S;
      R3', R4' and R5' are independently selected from H, F, Cl, Br, I and substituted or unsubstituted C₁ to C₁₀ alkyl, in particular Me and Et
      preferably X is S and R3' and R5' are independently selected from H and Me and R4' is H;
   R3 and R4 are independently selected from H, and unsubstituted or halosubstituted Me or Et, preferably, R3 and R4 are H;
   R5 is selected from H, F, Cl, Br, I and substituted or unsubstituted C₁ to C₁₀ alkyl, preferably R5 is H;
   or a pharmaceutically acceptable salt, solvate, tautomer or ester thereof.

In a preferred embodiment, the compound is a benzoxazole according to 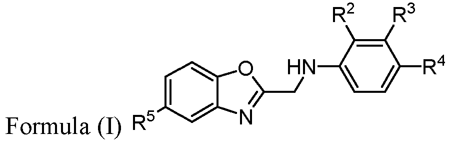 or a pharmaceutically acceptable salt, solvate, tautomer or ester thereof, wherein
R2 is phenyl according to 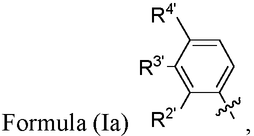 wherein
   R2' is selected from H, F, NH₂ and OMe,
   R3' is selected from H, F, NH₂, NHMe, NHPr,
   R4' is selected from H, NHMe
      or
R2 is heteroaryl according to 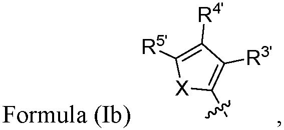 wherein
   X is S; and
   R3' and R5' are independently selected from H and Me,
   R4' is H,
      and
R3, R4 and R5 each are H.

In particularly preferred embodiments, the compound is a benzoxazole according to Formula (I) or a pharmaceutically acceptable salt, solvate, tautomer or ester thereof selected from the from the group consisting of

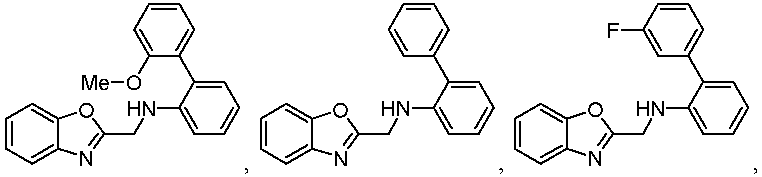

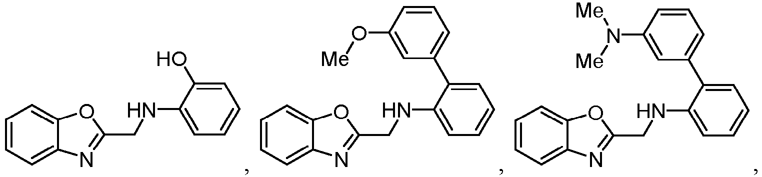

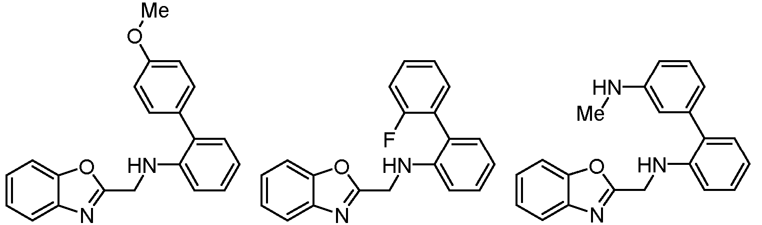

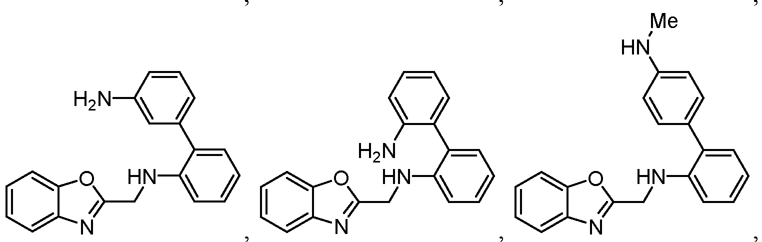

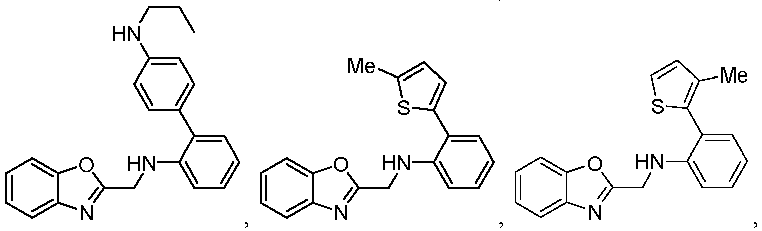

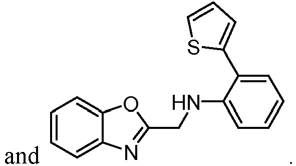

In a more preferred embodiment, the benzoxazole according to Formula (I) or a pharmaceuti-call acceptable salt, solvate, tautomer or ester thereof is selected from the group consisting of

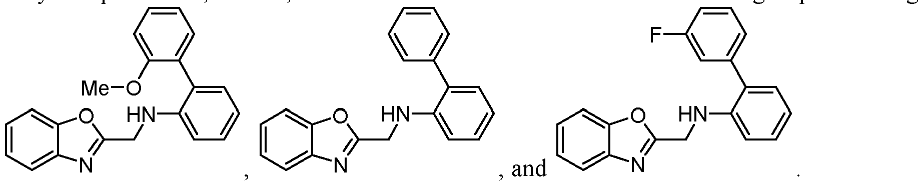

In a yet more preferred embodiment, the benzoxazole according to Formula (I) or a pharmaceutically acceptable salt, solvate, tautomer or ester thereof is 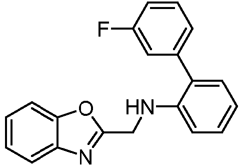 or

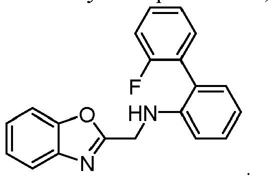

To the knowledge of the present inventors, benzoxazole 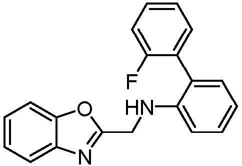 (also designated as CPD 353 herein ) is the most potent human TRPM4 antagonist to date with an IC50 of 83 nM.

### Anthranilic acid according to Formula (II)

In another embodiment, the compound is an anthranilic acid according to Formula (II) 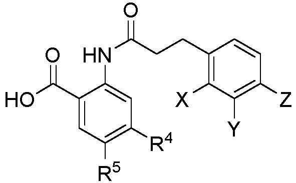 or a pharmaceutically acceptable salt, solvate, tautomer or ester thereof,
wherein
R4 and Z are independently selected from H and substituted or unsubstituted C₁ to C₁₀ alkyl, in particular CF₃;
R5, X and Y are independently selected from H, F, Cl, Br, I and halosubstituted or unsubstituted C₁ to C₁₀ alkyl, in particular CF₃,
or a pharmaceutically acceptable salt, solvate, tautomer or ester thereof.

Anthranilic acid compounds according to Formula (II) are characterized by the phenylpropane group as an accepted moiety for the amine group of the anthranilic acid. In some embodiments, anthranilic acid bears Br, Cl or CF₃ substituents at position 5. Unlike the known anthranilic acid ortholog CBA, anthranilic acid compounds according to the present invention do not accommodate chlorination at position 4, suggesting a distinct binding conformation.

Anthranilic acid compounds according to Formula (II) provide for steric flexibility. Advantageously, the anthranilic acid compounds according to Formula (II) provide for sub-micromolar IC50 values. This indicates a common binding site, with channel specificity modulated by the phenylpropane group of anthranilic acid compounds according to the invention.

In a preferred embodiment, in an anthranilic acid according to 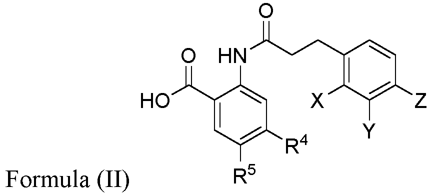 or a pharmaceutically acceptable salt, solvate, tautomer or ester thereof,
X and Y each are independently selected from Hand Cl,
R4 and Z each are H, and R5 is selected from Cl, Br and CF₃.

In more preferred embodiments, the compound is an anthranilic acid according to Formula (II) or a pharmaceutically acceptable salt, solvate, tautomer or ester thereof selected from the group consisting of

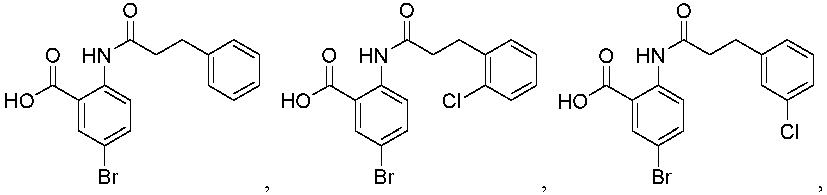

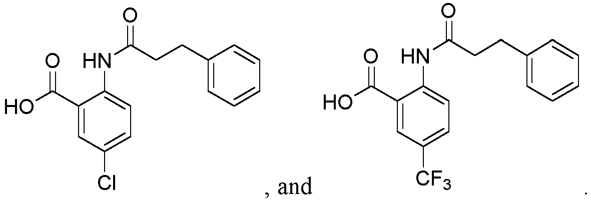

For example, the anthranilic acid compound 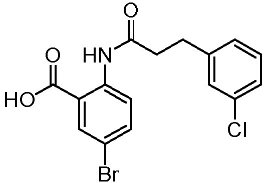 (also designated as CMP512 herein) which is within the scope of Formula (II) defining anthranilic acid compounds disclosed herein, demonstrates activity with nanomolar IC50 values not only on TRPM4 (SEQ ID NO: 1) but TRPM5 (SEQ ID NO: 4). This indicates a common binding site, with channel specificity modulated by the phenylpropane group of anthranilic acid compounds according to the invention.

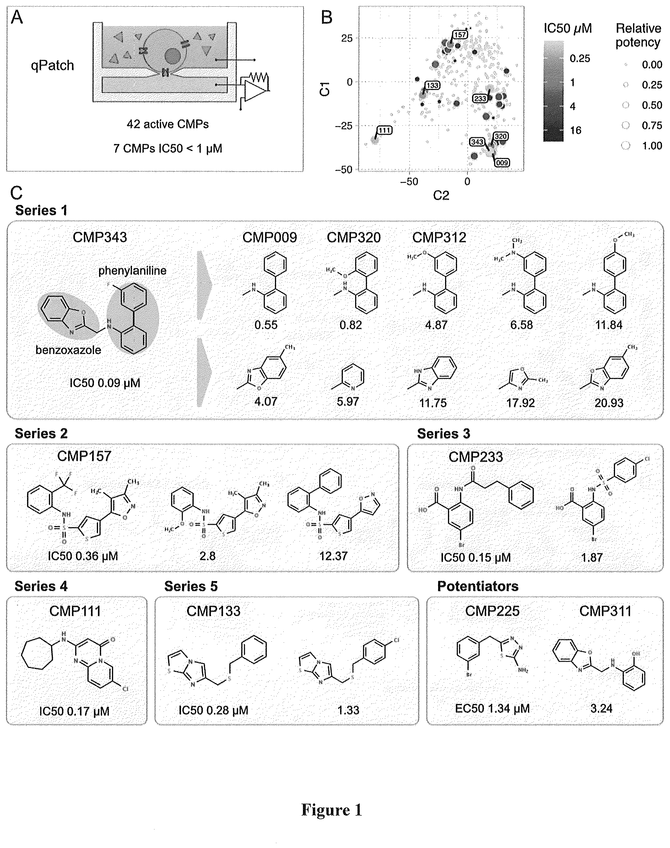

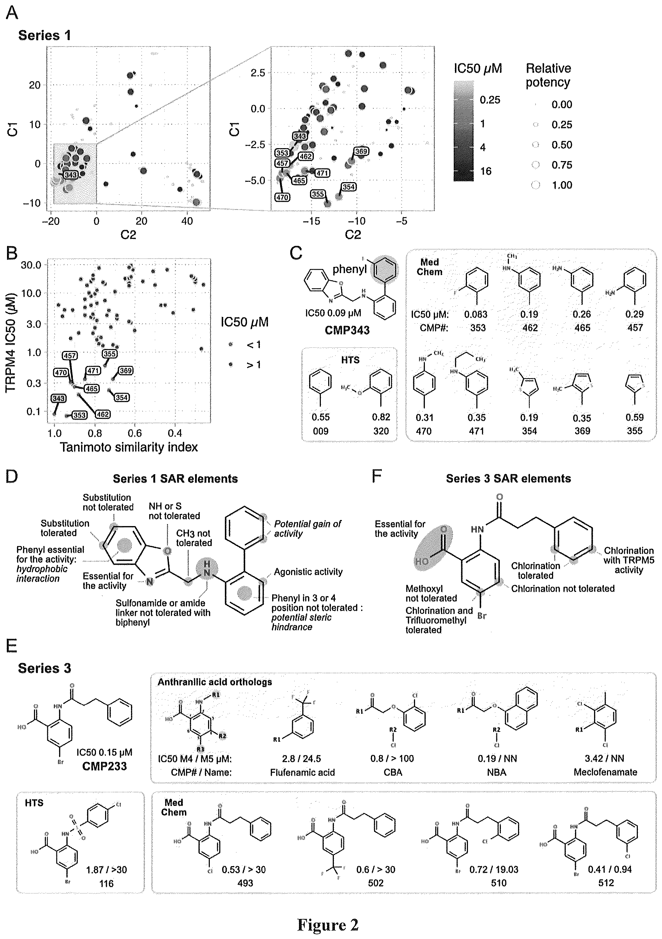

In an even more preferred embodiment, the anthranilic acid according to Formula (II) is 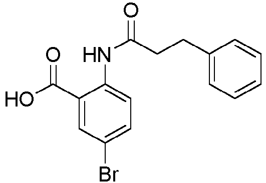 (also designated herein as "CMP233"), or a pharmaceutically acceptable salt, solvate, tautomer or ester thereof, which provides for an IC50 of 0.15 µM (see e.g. Figures 1C and 2E). CMP233 exhibits one of the highest affinities among anthranilic acid ortholog inhibitors, coupled with increased flexibility for subsequent structural modifications to improve its pharmacokinetic properties. Advantageously, *in vitro* pharmacological assessments indicate no off-target binding, and the bioavailability in the heart appears sufficient for conducting treatment studies in mouse models of TRPM4-related cardiac diseases ^{45,48}.

### 5-(5-Isoxazolyl)-2-thiophenesulfonamide according to Formula (III)

In another embodiment, the compound is a 5-(5-Isoxazolyl)-2-thiophenesulfonamide according to 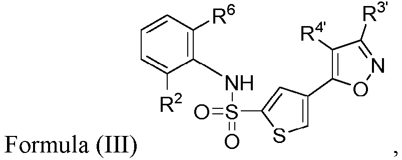 wherein
R2 is selected from Hand C₁ to C₁₀ alkyloxy groups, in particular methoxy, eth-oxy and propyloxy;
R6 is selected from H, F, Cl, Br, I, halosubstituted or unsubstituted C₁ to C₁₀ alkyl, in particular CF₃, and 5 to 10 membered non-annulated or annulated aryl or heteroaryl, in particular phenyl;
R3' and R4' are independently selected from H and substituted or unsubstituted C₁ to C₁₀ alkyl, in particular Me, Et, Pr, iPr and CF₃,
or a pharmaceutically acceptable salt, solvate, tautomer or ester thereof.

In a preferred embodiment, of the 5-(5-Isoxazolyl)-2-thiophenesulfonamide according to Formula (III), R3' and R4' both are Me. More preferably, the compound is a 5-(5-Isoxazolyl)-2-thiophenesulfonamide according to Formula (III) selected from the group consisting of

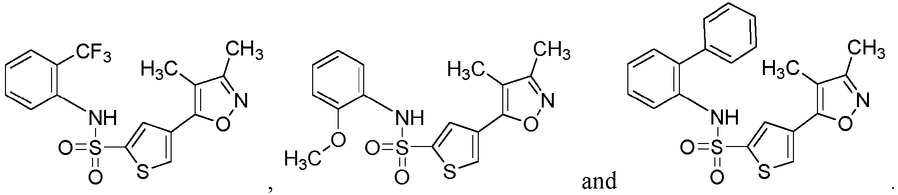

In a more preferred embodiment, the the 5-(5-Isoxazolyl)-2-thiophenesulfonamide according to

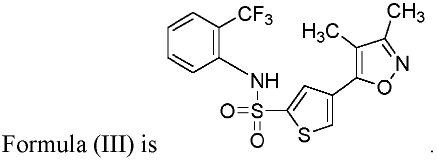

### Benzpyrimidine according to Formula (IV)

In another embodiment, the compound is a benzpyrimidine according to Formula (IV) 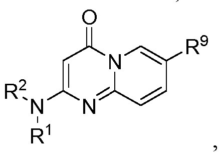 wherein
R1 and R2 are independently selected from Hand C₁ to C₈ alkyl and cycloalkyl, with R1 and R2 optionally forming a fused ring system; and
R9 is selected from H, F, Cl, Br, I, halosubstituted or unsubstituted C₁ to C₁₀ alkyl, in particular CF₃,
or a pharmaceutically acceptable salt, solvate, tautomer or ester thereof.

In a preferred embodiment of the benzpyrimidine according to Formula (IV) R1 is H and R2 is cycloheptyl and R9 is selected from H, F, Cl, Br, I and CF₃.

In a more preferred embodiment, the benzpyrimidine according to Formula (IV) is 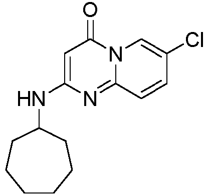 or a pharmaceutically acceptable salt, solvate, tautomer or ester thereof..

### Thiadiazole according to Formula (V)

In another embodiment, the compound is a thiadiazole according to Formula (V) 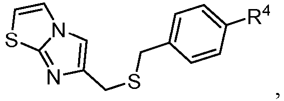
wherein R4 is selected from H, F, Cl, Br, I, halosubstituted or unsubstituted C₁ to C₁₀ alkyl, in particular CF₃,
or a pharmaceutically acceptable salt, solvate, tautomer or ester thereof.

In a preferred embodiment of the thiadiazole according to Formula (V) R4 is H or Cl.

In a more preferred embodiment, the thiadiazole according to Formula (V) is selected from the group consisting of 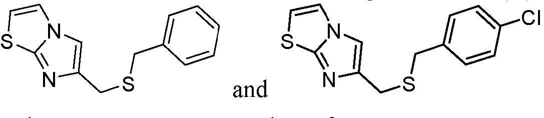 or a pharmaceutically acceptable salt, solvate, tautomer or ester thereof.

In a most preferred embodiment, the thiadiazole according to Formula (V) is 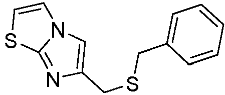 or a pharmaceutically acceptable salt, solvate, tautomer or ester thereof.

### Pharmaceutical compositions

The TRPM4-inhibitory compound of the present invention as described above will normally be provided in the form of a pharmaceutical composition which also comprises one or more excipients, carriers and/or diluents which are suitable for the intended way of administration. Generally, the compound may be administered in any suitable form that does not interfere with its TRPM4-inhibitory activity. The preferred route of administration will depend *inter alia* on the location of the neurodegeneration to be treated. For example, the compound may be administered orally in the form of tablets, capsules, granule, powder, liquids, and the like. Alternatively, the TRPM4-inhibitory compound may be formulated for being administered parenterally, e.g. by intravenous injection or intravenous infusion. In a preferred aspect, the TRPM4-inhibitory compound is administered to the subject by intravenous infusion, more preferably by short-term infusion within less than 60 min, e.g. within 30 min, 20 min or 15 min.

Compositions suitable for injection and/or infusion include solutions or dispersions and powders for the extemporaneous preparation of such injectable solutions or dispersions. The composition for injection must be sterile and should be stable under the conditions of manufacturing and storage. Preferably, the compositions for injection and/or infusion also include a preservative, such as a chlorobutanol, phenol, ascorbic acid, thimerosal, and the like. For intravenous administration, suitable carriers may comprise physiological saline, bacteriostatic water, Cre-mophor EL^{™} (BASF) or phosphate-buffered saline (PBS). Sterile solutions for injection and/or infusion can be prepared by incorporating the TRPM4-inhibitory compound in the required amount in an appropriate solvent followed by filter sterilization.

The pharmaceutical compositions of the present invention will comprise an amount of the TRPM4-inhibitory compound that is effective to inhibit the TRPM4 ion channel, thereby reducing the extent of neurodegeneration by protecting the NS, preferably the CNS, from neuronal loss. The therapeutically effective amount of the TRPM4-inhibitory compound to be administered will depend on several parameters, such as the mode of administration, the particular TRPM4-associated disorder to be treated, the severity of the disease, the history of the disease, the age, height, weight, health, and physical condition of the individual to be treated, and the like. A therapeutically effective amount of the TRPM4-inhibitory compound can be determined by one of ordinary skill in the art without undue experimentation given the disclosure set forth herein.

The TRPM4-inhibitory compound will preferably be administered to a subject in an amount that ranges from 0.1 µg/kg body weight to 10,000 µg/kg body weight, e.g. from 0.5 µg/kg to 7,500 µg/kg body weight, from 1.0 µg/kg to 5,000 µg/kg body weight, from 5.0 µg/kg to 3,000 µg/kg body weight, from 7.5 µg/kg to 2,500 µg/kg body weight, from 10 µg/kg to 2,000 µg/kg body weight, from 25 µg/kg to 1,500 µg/kg body weight, from 50 µg/kg to 1,000 µg/kg body weight, from 100 µg/kg to 800 µg/kg body weight, from 300 µg/kg to 600 µg/kg body weight, and more preferably from 400 µg/kg to 500 µg/kg body weight.

For compositions for oral administration (e.g. tablets and capsules), the term "carrier" includes vehicles such as common excipients e.g. binding agents, for example syrup, acacia, gelatin, sorbitol, tragacanth, polyvinylpyrrolidone (Povidone), methylcellulose, ethylcellulose, sodium carboxymethylcellulose, hydroxypropylmethylcellulose, sucrose and starch; fillers and carriers, for example corn starch, gelatin, lactose, sucrose, microcrystalline cellulose, kaolin, mannitol, dicalcium phosphate, sodium chloride and alginic acid; and lubricants such as magnesium stearate, sodium stearate and other metallic stearates, glycerol stearate, stearic acid, silicone fluid, talc waxes, oils and colloidal silica. Flavouring agents such as peppermint, oil of wintergreen, cherry flavouring and the like can also be used. It may be desirable to add a colouring agent to make the dosage form readily identifiable. Tablets may also be coated by methods well known in the art.

A tablet may be made by compression or moulding, optionally with one or more accessory ingredients. Compressed tablets may be prepared by compressing in a suitable machine the compound in a free-flowing form such as a powder or granules, optionally mixed with a binder, lubricant, inert diluent, preservative, surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active agent.

Other formulations suitable for oral administration include lozenges comprising the active agent in a flavoured base, usually sucrose and acacia or tragacanth; pastilles comprising the active agent in an inert base such as gelatin and glycerin, or sucrose and acacia; and mouthwashes comprising the active agent in a suitable liquid carrier.

Parenteral formulations will generally be sterile.

Apart from the TRPM4-inhibitory compound, the pharmaceutical composition provided by the present invention may further comprise at least one additional pharmaceutically active compound which is useful in treating or preventing a TRPM4-associated disorder in a subject such as other anti-neurodegenerative or anti-inflammatory compounds which are commonly used in the treatment of neurodegenerative diseases, e.g. interferon beta-1a, interferon beta-1b, fam-pridine, fingolimod hydrochloride, natalizumab, glatiramer acetate, or mitoxantrone. Where the TRPM4-inhibitory compound is used in combination with another anti-neurodegenerative agent, the two active ingredients can be administered to the subject in the form of a single pharmaceutical composition comprising both agents and pharmaceutically acceptable excipients and carriers. Administration of such a pharmaceutical composition will automatically result in a simultaneous administration of both agents. Alternatively, the two therapeutic agents may also be administered separately from each other, i.e. in the form of two separate pharmaceutical compositions, one containing the TRPM4-inhibitory compound, and the other containing the additional anti-neurodegenerative or anti-inflammatory agent. The two separate compositions can be administered simultaneously, i.e. at the same time at two distinct sites of administration, or they may be administered sequentially (in either order) to the same site or to different sites of administration.

The precise amount of a composition as defined herein which is therapeutically effective, and the route by which such compound is best administered, is readily determined by one of ordinary skill in the art. Such amounts will depend, of course, on the particular condition being treated, the severity of the condition, the individual patient parameters including age, physical condition, size and weight, the duration of the treatment, the nature of concurrent therapy (if any), the specific route of administration and like factors within the knowledge and expertise of the health practitioner. These factors are well known to those of ordinary skill in the art and can be addressed with no more than routine experimentation. It is generally preferred that a maximum dose of the individual components or combinations thereof be used, that is, the highest safe dose according to sound medical judgment. It will be understood by those of ordinary skill in the art, however, that a patient may insist upon a lower dose or tolerable dose for medical reasons, psychological reasons or for virtually any other reasons.

The doses of the compound or composition according to the invention administered to a subject can be chosen in accordance with different parameters, in particular in accordance with the mode of administration used and the state of the subject. Other factors include the desired period of treatment. In the event that a response in a subject is insufficient at the initial doses applied, higher doses (or effectively higher doses by a different, more localized delivery route) may be employed to the extent that patient tolerance permits.

Preferably, both the composition comprising the TRPM4-inhibitory compound and the composition comprising the second anti-neurodegenerative or anti-inflammatory agent are administered according to a weekly dosing regimen, more preferably a regimen in which a single dose of the TRPM4-inhibitory compound and a single dose of the anti-neurodegenerative or anti-inflammatory agent is administered every week for a treatment period of 2 or more weeks, for example, 3, 4, 5, 6, 7, 8, 9, 10, 11, 12, 13, 14, 15, 16, 17, 18, 19, 20, or more weeks. Preferably, the treatment period comprises at least 12 weeks. It will also be possible to administer the overall weekly dose of the TRPM4-inhibitory compound and/or the anti-neurodegenerative or anti-inflammatory agent in more than one administration per week, e.g. in 2 or 3 administrations per week. In a preferred embodiment, the amount of the TRPM4-inhibitory compound to be administered weekly is delivered as a single intravenous infusion per week, and the amount of the anti-neurodegenerative or anti-inflammatory agent to be administered weekly is also given as a single intravenous infusion per week, either at the same day of administration of the TRPM4-inhibitory compound, e.g. within about 10 minutes to about 6 hours after administration of the TRPM4-inhibitory compound has been completed, more preferably within about 30, 60, 90, 120, 150, 180, 210 or 240 minutes, or at any of days 2, 3, 4, 5, 6 or 7 of the week.

For example, a combination therapy with the above-mentioned agents that is based on a weekly dosing regimen begins on day 1 of a treatment period, and a first therapeutically effective dose of the TRPM4-inhibitory compound is administered at that day. A first therapeutically effective dose of the additional anti-neurodegenerative or anti-inflammatory agent can be administered either on the same day, e.g. simultaneously or within about 30, 60, 90, 120, 150, 180, 210 or 240 minutes after administration of the TRPM4-inhibitory compound. Alternatively, the additional anti-neurodegenerative or anti-inflammatory agent can be administered at any of days 2, 3, 4, 5, 6 or 7 of the first week. At day 8, a second therapeutically effective dose of the TRPM4-inhibitory compound is administered accompanied or followed by the second administration of the additional anti-neurodegenerative or anti-inflammatory agent. The skilled person will readily be able to design other administration regimens which are suitable for the delivery of the combined active ingredients.

### DESCRIPTION OF THE FIGURES

**Figure 1** shows the identification of compounds disclosed herein, i.e. benzoxazoles according to Formula (I) (also referred to herein as "Series 1"), anthranilic acids according to Formula (II) (also referred to herein as "Series 3"), 5-(5-Isoxazolyl)-2-thiophenesulfonamides according to Formula (III) (also referred to herein as "Series 2"), benzpyrimidines according to Formula (IV) (also referred to herein as "Series 4"), and thiadiazoles according to Formula (V) (also referred to herein as "Series 5") by high-throughput screening. (A) Schematic illustration of the high-throughput screening (HTS) workflow for the identification of TRPM4 antagonists. The HTS involved screening a library comprising 256,286 small molecules. Active compounds were identified using a kinetic FLIPR assay based on membrane-potential sensitive dyes followed by subsequent validation of their activity and determination of IC50 values with an automated patch clamp system (QPatch). (B) Multi-Dimensional Scaling (MDS) analysis presenting the distribution of 357 compounds identified during the high-throughput screening. The respective IC50 values determined by QPatch measurements are represented, with compounds exhibiting IC50 values below ≤1 µM highlighted. (C) Chemical features of representative compounds disclosed herein i.e. of benzoxazoles according to Formula (I) (also referred to herein as "Series 1"), anthranilic acids according to Formula (II) (also referred to herein as "Series 3"), 5-(5-Isoxazolyl)-2-thiophenesulfonamides according to Formula (III) (also referred to herein as "Series 2"), benzpyrimidines according to Formula (IV) (also referred to herein as "Series 4"), and thiadiazoles according to Formula (V) (also referred to herein as "Series 5").
**Figure 2** shows the medicinal chemistry of representative compounds disclosed herein and structure-activity relationship. A) Multi-Dimensional Scaling (MDS) analysis of illustrative benzoxazoles according to Formula (I) with IC50 values colour coded for 78 active compounds. (B) Illustration of the correlation between lead similarity and IC50. (C) Benzoxazoles according to Formula (I) with IC50 values below 1 µM. The focus lies on modifications of the phenyl residue that maintain IC50s in the nanomolar range. (D) Structure-Activity Relationship (SAR) of benzoxazoles according to Formula (I). (E) Comparison of anthranilic acid compounds according to Formula (II) disclosed herein with known TRPM4 antagonists of the anthranilic acid ortholog family, i.e. flufenamic acid, CBA, NBA and meclofenamate. (F) SAR elements of anthranilic acids according to Formula (II).

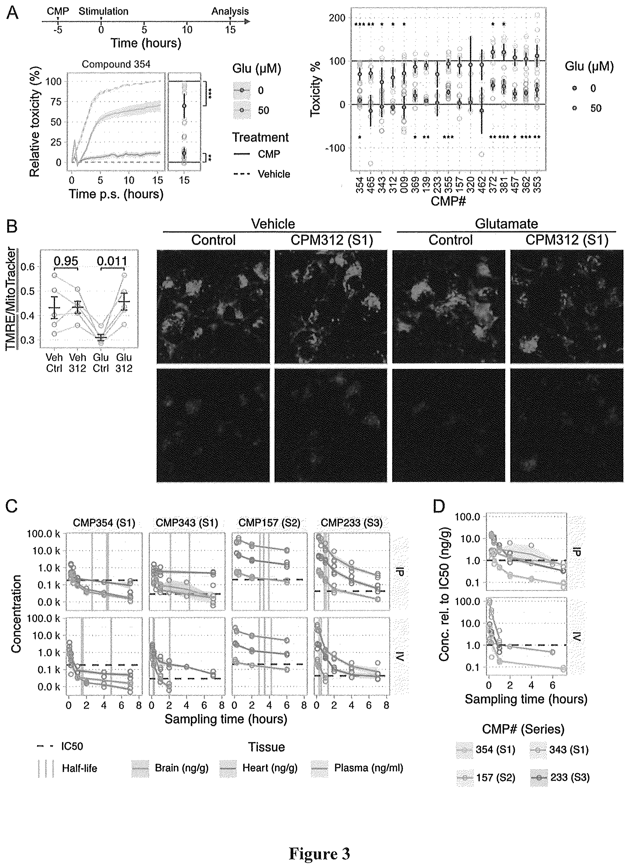

**Figure 3** shows the results of neuroprotective *in vitro* evaluation and *in vivo* pharmacokinetics of compounds disclosed herein. (**A**) *In vitro* assessment of compound activity on glutamate-induced excitotoxicity in mature primary neuronal cultures. Neuronal cultures were treated with 5 µM of the specified compound or vehicle control 5 hours prior to glutamate stimulation, assessing compound rescue activity and toxicity relative to minimal (Vehicle + 0 µM Glu) and maximal (Vehicle + 50 µM Glu) excitotoxicity. Time course of a representative benzoxazole compound according to Formula (I), 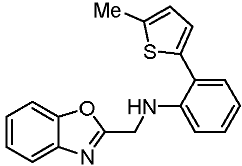 (also referred to herein as "CPD 354) with virtual endpoint at 15 hours post stimulation and assay window definition (left). Toxicity of compound treated cultures with and without glutamate stimulation (right). Single biological replicates, mean and 95% confidence interval are shown; *n* ≥ 4; **P* < 0.05, ***P* < 0.01, ****P* < 0.001; One-sample Wilcoxon signed-rank test. (**B**) Assessment of benzoxazole compound according to Formula (I), 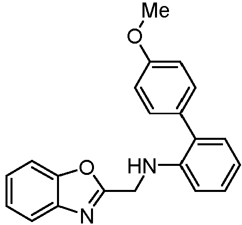 (also referred to herein as "CMP312") on mitochondrial integrity after glutamate-induced excitotoxicity. Mitochondrial membrane potential was measured by the mean fluorescence intensity of TMRE normalized to MitoTracker; *n* = 5; Paired *t*-test. (**C**) *In vivo* pharmacokinetics of representative compounds disclosed herein, i.e. two benzoxazole compounds according to Formula (I), 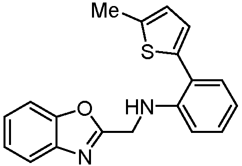 (also referred to herein as "CMP354") and 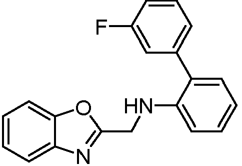 (also referred to herein as "CMP343"), an anthranilic acid according to Formula (II), 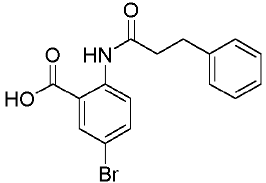 (also referred to herein as "CMP233"), and a 5-(5-Isoxazolyl)-2-thiophenesulfonamide according to 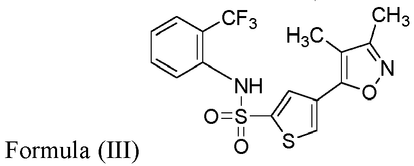 (also referred to herein as "CMP157"). Time course of compound concentration in the brain, heart, and plasma after a single dose via intraperitoneal (i.p.) or intravenous (i.v.) application. Half-life estimates by non-compartmental analysis are shown as vertical line and IC50 value as dashed horizontal line. (**D**) Compound concentration in the brain relative to respective IC50 concentrations after a single dose via intra-peritoneal or intra-venous routes.

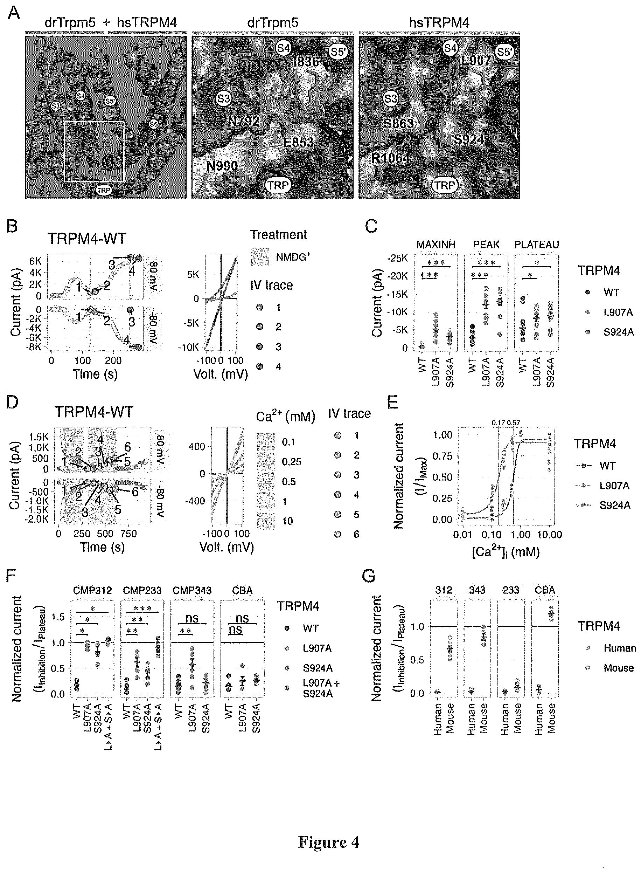

**Figure 4** shows the results of the electrophysiological analysis of proposed compound binding site at TRPM4 (SEQ ID NO: 1). (A) Proposed compound binding site. (left) Aligned superimposition of drTRPM5 (SEQ ID NO: 5) structure (blue) in complex with its antagonist NDNA (green) and hsTRPM4 (SEQ ID NO: 1) (magenta). Transmembrane helices (S) and the TRP domain are labeled. drTrpm5 binding pocket of NDNA (middle) and the corresponding pocket in hsTRPM4 (right). Divergent amino acids are labeled. (B, C) Whole cell voltage-clamp analysis of basic electrophysiological properties of HEK293T cells expressing wild-type (WT, SEQ ID NO: 1) or mutant hsTRPM4 (SEQ ID NO: 6 and 7, respectively). (B) Representative time course of current levels at -80 and +80 mV recorded in hsTRPM4-expressing HEK293T cells. TRPM4 currents were elicited upon patch rupture by 100 µM CaCl₂, loaded in the pipette solution. Extracellular Na⁺ was replaced by equimolar N-methyl-D-glucamine (NMDG⁺) to identify TRPM4 currents. Individual current-to-voltage relationship measured in the same hsTRPM4-expressing HEK293T cells are shown next to the representative time course. IV trace numbers indicate the respective time points of the measurement to calculate peak current, maximal inhibition and plateau current. (C) Quantification of the peak current, maximal inactivated current and the steady-state plateau current between WT (SEQ ID NO: 1) and hsTRPM4-L907A (SEQ ID NO: 6) and hsTRPM4-S924A (SEQ ID NO: 7) mutants. (D, E) Assessment of Ca²⁺ sensitivity of WT (SEQ ID NO: 1) and mutant hsTRPM4 (SEQ ID NO: 6 and 7, respectively). (D) Representative time course of current levels at -80 mV and +80 mV recorded in hsTRPM4-expressing HEK293T cells using the inside-out patch clamp technique. TRPM4 currents were activated by 500 µM Ca²⁺ and deactivated by 10 mM EGTA. Individual current-to-voltage relationship measured in the same hsTRPM4-expressing HEK293T cell are shown next to the representative time course. (E) The Ca²⁺ concentration-to-current relationship of WT (SEQ ID NO: 1) and hsTRPM4-L907A and hsTRPM4-S924A mutants (SEQ ID NO: 6 and 7, respectively). Likelihood maximization was used to estimate parameter for the sigmoidal fitting curves with y-maximum, slope and midpoint of 0.94, 8.05 and 0.55 (WT); 0.90, 18.00 and 0.16 (L907A); 0.91 and 17.57 (S924A). (F) Quantification of relative inhibition in whole cell voltage-clamp configuration of WT and mutant hsTRPM4 by representative compounds disclosed herein, namely benzoxazole compounds according to Formula (I), 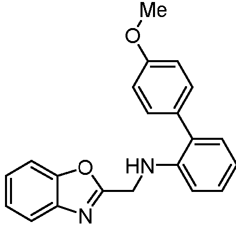 and 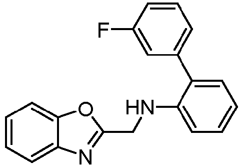 (also referred to herein as "CMP312", and "CMP343"), anthranilic acid according to Formula (II), 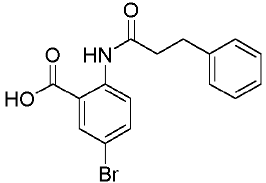 (also referred to herein as "CMP233"), and CBA. (G) Comparison of relative inhibition in whole cell voltage-clamp configuration between mouse and human TRPM4. C: *n* ≥ 10; F: *n* ≥ 5; **P* < 0.05, ***P* < 0.01, ****P* < 0.001; Wilcoxon rank-sum test.

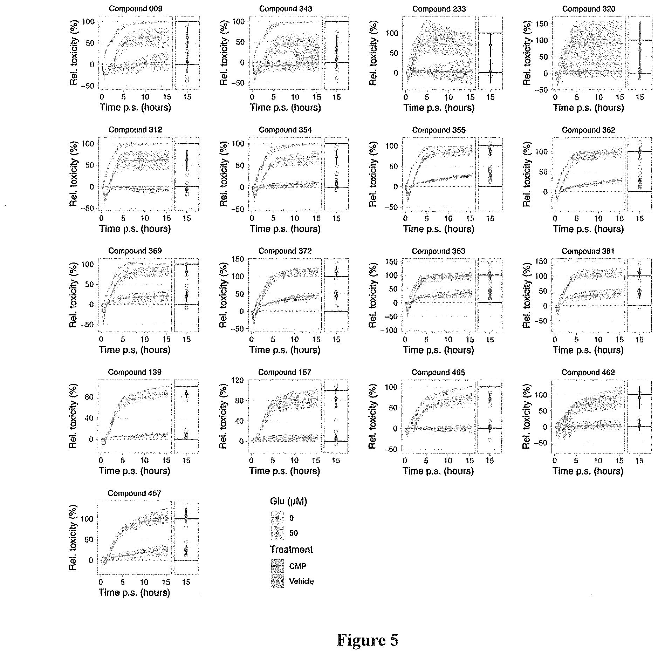

**Figure 5** shows the results of the neuroprotective *in vitro* evaluation. *In vitro* evaluation of compound activity on glutamate-induced excitotoxicity in mature primary neuronal cultures. Time course of all tested compounds.

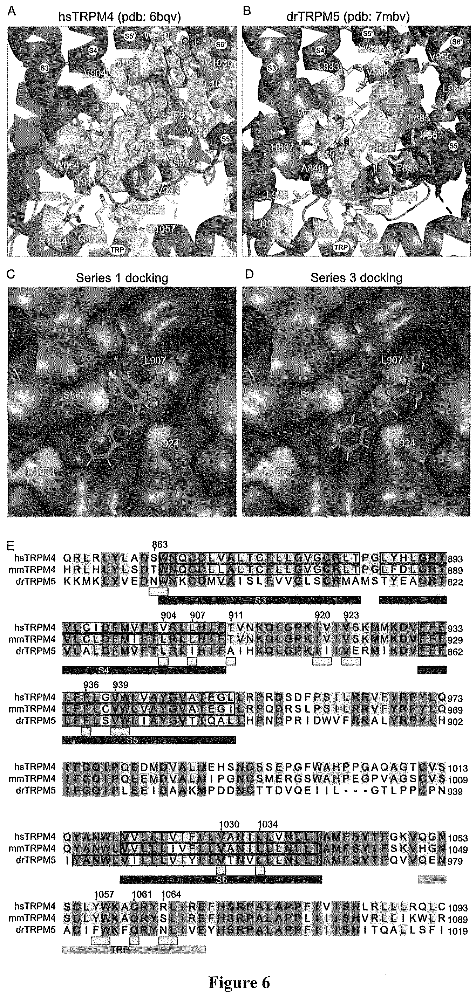

**Figure 6** shows the proposed binding side in hsTRPM4 (SEQ ID NO: 1) and drTRPM5 (SEQ ID NO: 5) of compounds disclosed herein. (A, B) The proposed compound binding site in hsTRPM4 (A) and the homologous site in drTRPM5 (B) are depicted. Transmembrane helices S3-S5, the S4-S5 linker, TRP domain, and helices S5' and S6' from the neighboring TRPM4 subunit, along with the amino acids lining the binding pocket, are labeled. Predicted pocket volumes are represented as colored clouds, with major differentiating amino acids between hsTRPM4 and drTRPM5 highlighted in red. (C, D) Docking of a representative benzoxazole according to Formula (I), 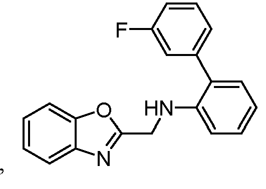 (also referred to herein as "CMP343") (C) and a representative anthranilic acid according to Formula (II), Series 3 Compound 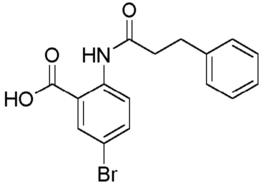 (also referred to herein as "CMP233") (D) to the proposed binding site. (E) Sequence alignment of hsTRPM4 (SEQ ID NO: 1), mmTRPM4 (SEQ ID NO: 8) and drTRPM5 (SEQ ID NO: 5) in the region of the proposed binding site. Relevant amino acids from figure A are labeled in yellow, transmembrane helices are labeled in black and the TRP domain in gray.

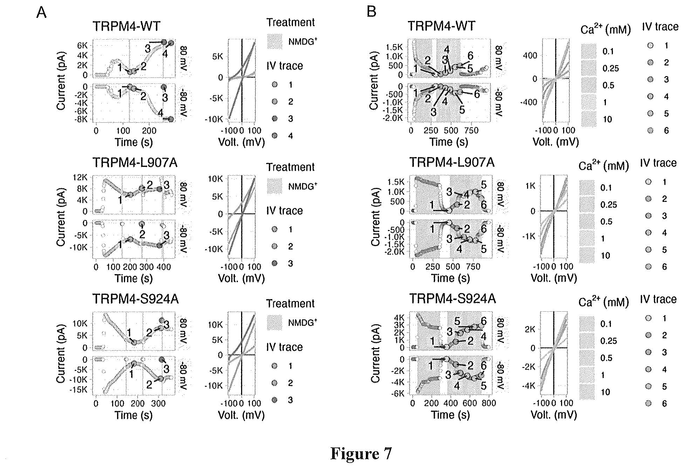

**Figure 7** shows the results of the electrophysiological characterization of TRPM4 mutants. (A) Whole cell voltage-clamp analysis of basic electrophysiological properties of HEK293T cells expressing wild-type or mutant TRPM4. Representative time course of current level at -80 and +80 mV measured in either hsTRPM4-WT (SEQ ID NO: 1), hsTRPM4-L907A (SEQ ID NO: 6) or hsTRPM4-S924A (SEQ ID NO: 7) expressing HEK293T cell using the whole cell patch clamp technique. TRPM4 currents were activated upon patch rupture by 100 µM CaCl₂, loaded in the pipette solution. Extracellular Na⁺ was replaced by equimolar NMDG⁺ to identify TRPM4 currents. Individual current-to-voltage relationship measured in either the same hsTRPM4-WT (SEQ ID NO: 1), hsTRPM4- L907A (SEQ ID NO: 6) or hsTRPM4-S924A (SEQ ID NO: 7) expressing HEK293T cell are shown next to the representative time course. (B) Determination of Ca²⁺ sensitivity of wild type and mutant TRPM4. Representative time course of current level at -80 and +80 mV measured in either a hsTRPM4-WT (SEQ ID NO: 1), hsTRPM4-L907A (SEQ ID NO: 6) or hsTRPM4-S924A (SEQ ID NO: 7) containing membrane patch using the inside-out patch clamp technique. TRPM4 currents were activated by 500 µM Ca²⁺ and deactivated by 10 mM EGTA. Individual current-to-voltage relationship measured in either the same hsTRPM4-WT (SEQ ID NO: 1), hsTRPM4-L907A (SEQ ID NO: 6) or hsTRPM4-S924A (SEQ ID NO: 7) expressing HEK-293T cell are shown next to the representative time course.

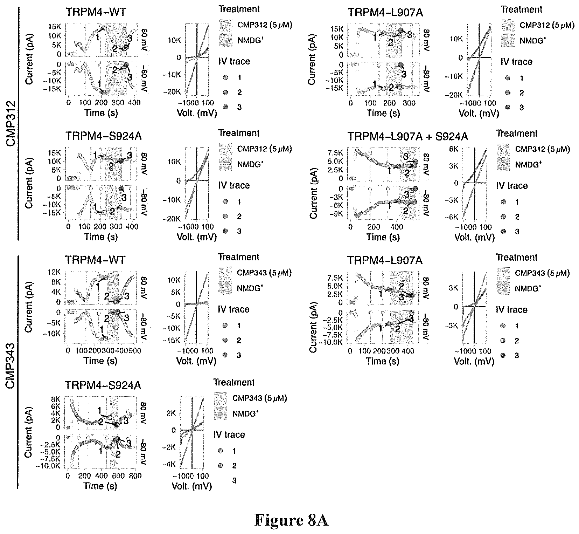

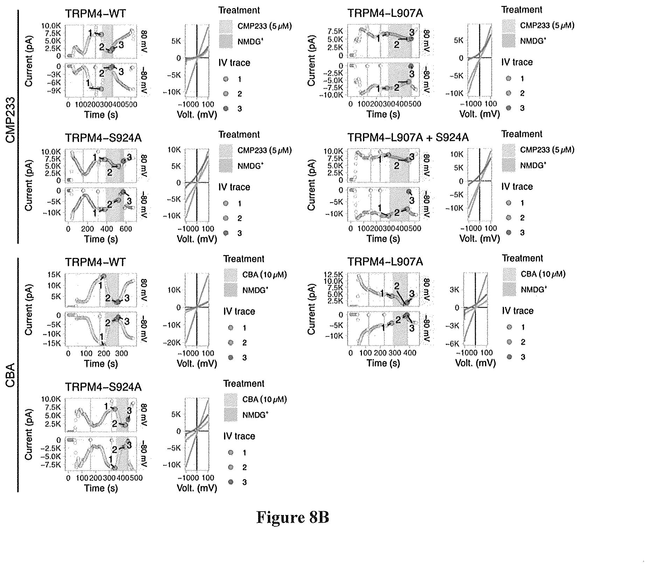

**Figures 8A** **and** **8B** show the results of the electrophysiological analysis of the proposed compound binding site. Whole cell voltage-clamp measurements of relative inhibition of wild-type (SEQ ID NO: 1 and mutant TRPM4 (SEQ ID NOs: 6 and 7, respectively) by benzoxazole compounds according to Formula (I), 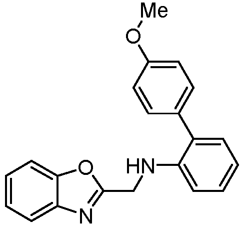 and 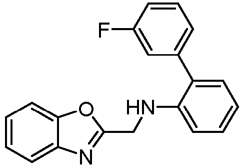 also referred to herein as "CMP312" and "CMP343", respectively), anthranilic acid compound according to Formula (II), 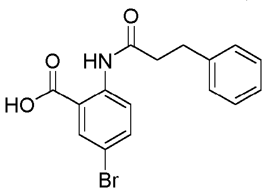 (also referred to herein as "CMP233") and CBA. Representative time course of current levels at -80 and +80 mV measured in either hsTRPM4-WT (SEQ ID NO: 1), hsTRPM4-L907A (SEQ ID NO: 6) or hsTRPM4-S924A (SEQ ID NO: 7) expressing HEK293T cell using the whole cell patch clamp technique. TRPM4 currents were activated upon patch rupture by 100µM CaCl₂, loaded in the pipette solution. Extracellular Na⁺ was replaced by equimolar NMDG⁺ to identify TRPM4 currents. Individual current-to-voltage relationship measured in either the same hsTRPM4-WT (SEQ ID NO: 1), hsTRPM4-L907A (SEQ ID NO: 6) or hsTRPM4-S924A (SEQ ID NO: 7) expressing HEK293T cell are shown next to the representative time course.

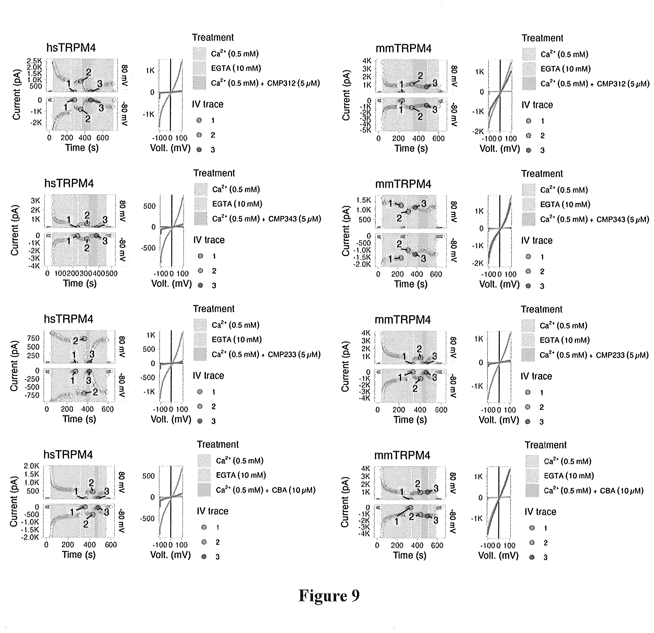

**Figure 9** shows the results of the electrophysiological comparison of human and mouse TRPM4. Whole cell voltage-clamp measurements of relative inhibition by benzoxazole compound according Formula (I), 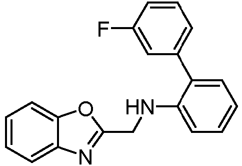 (also referred to herein as "CMP343"), and anthranilic acid compound according to Formula (II) 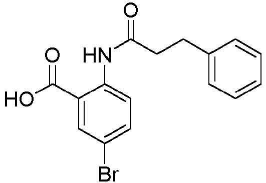 (also referred to herein as "CMP233") and CBA comparing mouse (mm) TRPM4 (SEQ ID NO: 8) and human TRPM4 (SEQ ID NO: 1). Representative time course of current levels at -80 and +80mV measured in either a hsTRPM4 (SEQ ID NO: 1)or mmTRPM4 (SEQ ID NO: 8) containing membrane patch using the inside-out patch clamp technique. TRPM4 currents were elicited by 500 µM Ca²⁺ and deactivated by 10 mM EGTA. Individual current-to-voltage relationship measured in either the same hsTRPM4 or mmTRPM4 expressing HEK293T cell are shown next to the representative time course.

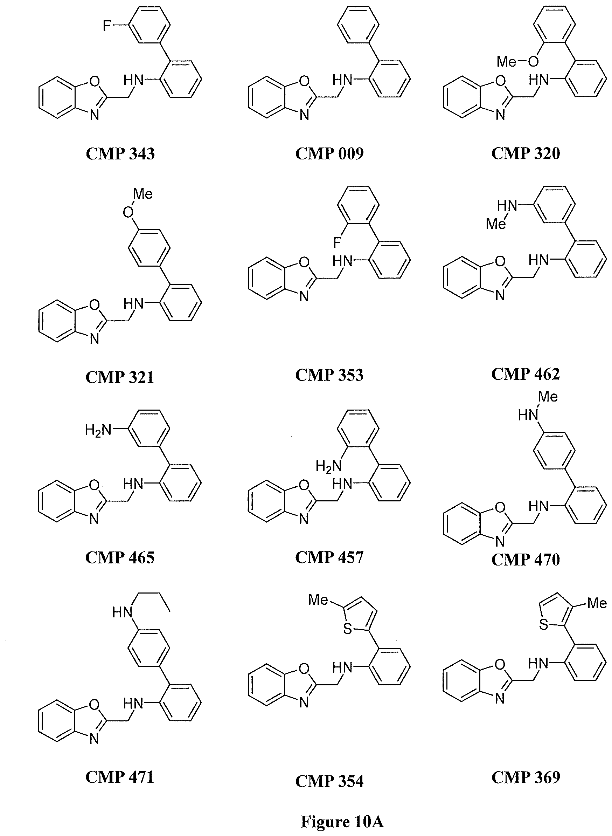

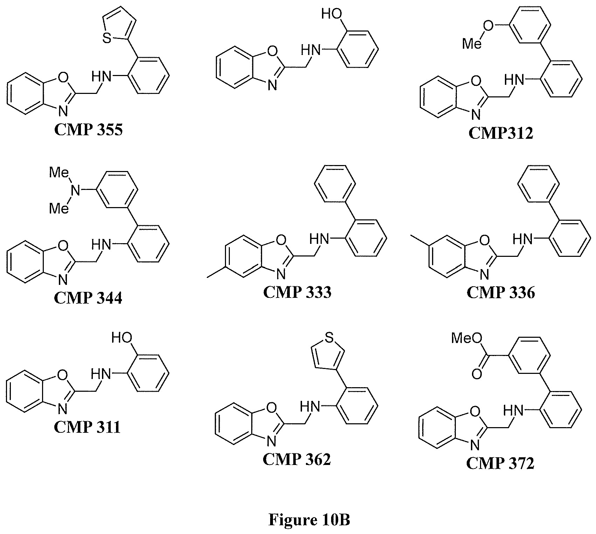

**Figures 10A** **and** **10B** show representative compounds of benzoxazole according to Formula (I) disclosed herein. Designations "CMP (...)" as used herein are indicated for the compounds where applicable.

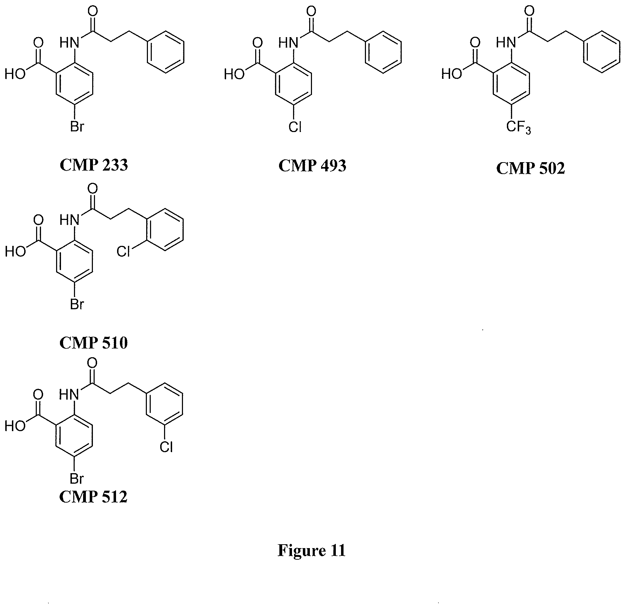

**Figure 11** shows representative compounds of anthranilic acid according to Formula (II) disclosed herein. Designations "CMP (...)" as used herein are indicated for the compounds where applicable.

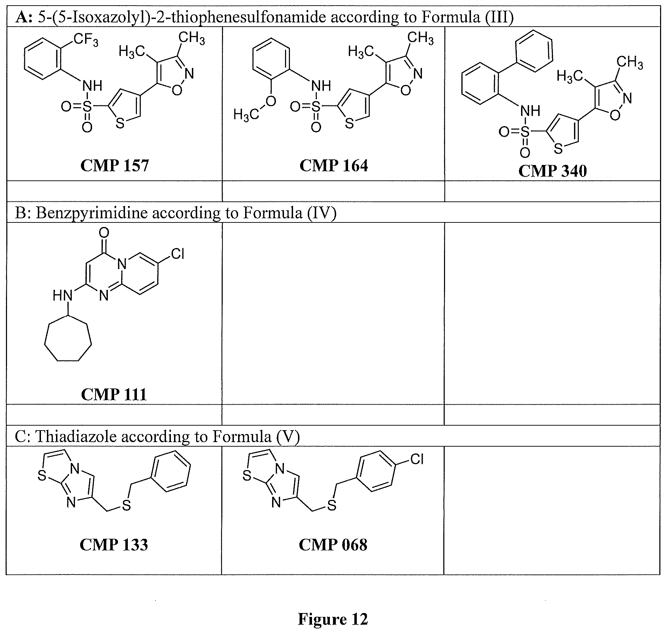

**Figure 12** shows representative compounds of 5-(5-Isoxazolyl)-2-thiophenesulfonamide according to Formula (III), benzpyrimidine according to Formula (IV), and thiadiazole according to Formula (V) disclosed herein. Designations "CMP (...)" as used herein are indicated for the compounds where applicable.

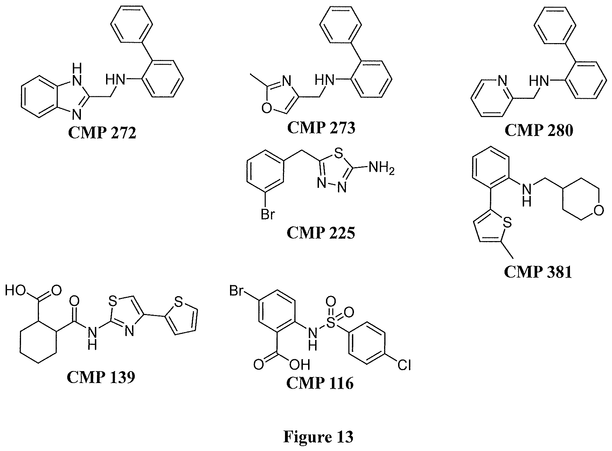

**Figure 13** shows further compounds also disclosed herein. Designations "CMP (...)" as used herein are indicated for the compounds where applicable.

### EXAMPLES

### Example 1: Assessment of IC50 values using the QPatch assay

For example, the anthranilic acid compound 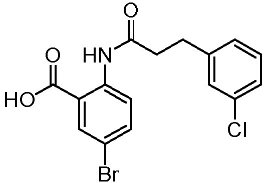 (also designated as CPD512 herein) which is within the scope of Formula (II) defining anthranilic acid compounds disclosed herein, demonstrates activity with nanomolar IC50 values not only on TRPM4 (SEQ ID NO: 1) but TRPM5 (SEQ ID NO: 4). This indicates a common binding site on TRPM4 and TRPM5, with channel specificity modulated by the phenylpropane group of anthranilic acid compounds according to the invention. IC50 values of representative compounds disclosed herein are summarized in Figures 1, 2 as well as Tables 1 and 4.

### Example 2: In vitro assays for determining ADMET parameters

*In vitro* assays were used to test absorption, distribution, metabolism, excretion and toxicity (ADMET) parameters for selected compounds. Compound absorption was assessed using parallel artificial membrane permeability (PAMPA) and Caco-2 cell permeability assays (cut-off criterion > 2 × 10⁻⁶ cm s⁻¹). Compound toxicity was evaluated by measuring inhibition of cytochrome P450 enzymes (CYP), the potassium channel hERG, and TRPM5, with all parameters meeting acceptable criteria (Table 1). To assess potential off-target activity of the compounds that could be relevant for safety in humans, *in vitro* pharmacology tests using the commercially available and widely used SafetyScreen44 interaction panel⁶⁰ covering eight protein families were conducted (Tables 2 and 3). The lack of off-target activities on hERG channels and *in vitro* pharmacology profiles are promising and consistent with absence of toxicity during *in vivo* pharmacokinetic assessments, see below.

**Table 1. In vitro ADMET profile of Series 1 compounds.**

| **CID** | **TRP M4 IC50** | **Inhib. at highest conc.** | **TRP M5 IC50** | **hERG IC50** | **Hep. clearance** | **Microsomes (m) clearance** | **Microsomes (h) clearance** | **Cyp IC50** | **Caco-2 perm.** | **PAMPA perm.** |
|---|---|---|---|---|---|---|---|---|---|---|
| | **µM** | **%** | **µM** | **µM** | **L/h/kg** | **L/h/kg** | **L/h/kg** | **µM** | **10⁻⁶ cm/s** | **10⁻⁶ cm/s** |
| **CMP343** | 0.09 | 48.76 | >10 | >30 | 19.3 | >54 | 34.2 | 2C9=32.2, 2D6>50, 3A4>50, 1A2=3.2, 2C19=3.5, TDI 3A4>50 | | |
| **CMP353** | 0.08 | 12.46 | >10 | >10 | 33.7 | >54 | 36.3 | 2C9=25.9, 2D6>50, 3A4>29.6, 1A2=1, 2C19=3.3 | | low sensitivity |
| **CMP354** | 0.19 | 12.2 | >10 | >10 | 17.6 | >54 | 27.6 | 2C9=25, 2D6>50, 3A4>50, 1A2=2.4, 2C19=3.7, TDI 3A4>50 | | |
| **CMP366** | 0.59 | 22.74 | >10 | >10 | 17.6 | >54 | 33.5 | 2C9=14.9, 2D6>50, 3A4>50, 1A2=1.3, 2C19=6.2, TDI 3A4>50 | | |
| **CMP369** | 0.35 | 24.24 | >10 | >10 | 20.8 | >54 | 36 | 2C9=25,4 2D6>50 , 3A4>50 , 1A2=27,2 2C19=11,4 | 52 | |
| **CMP457** | 0.29 | 24.69 | >30 | 24.91 | | 54 | 14.6 | 2C9=21.3 2D6>50 , 3A4=10.6 , 1A2=37.8 2C19=6.8 | 210 | 37.4 |
| **CMP462** | 0.19 | 39.14 | >30 | 8.27 | | 54 | 18.12 | 2C9=18 2D6>50 , 3A4=19.2 , 1A2=3.3 2C19=4 ) | 233 | 25 |
| **CMP465** | 0.26 | 23.36 | >30 | 26.57 | | 236 | 12.96 | 2C9=17.6 2D6>50 , 3A4=4.5 , 1A2=2.3 2C19=3.2 | 189 | 379 |
| **CMP470** | 0.31 | 79.9 | >30 | 10.6 | | 54 | 13.5 | 2C9=9.8 2D6>50 , 3A4=2.9 , 1A2=1.3 2C19=1.2 | | 22 |
| **CMP471** | 0.35 | 89.7 | >30 | 2.55 | | | | 2C9=17.2 2D6>50 , 3A4>50 , 1A2=16.7 2C19=14.7 | | no detection |

**Table 2. In vitro pharmacology profile of representative compounds disclosed herein, i.e. of benzoxazoles according to Formula (I) (CMP 343 and 354). Only inhibitions above 50% relative to the reference compound are shown and considered active.**

| **CID** | **Assay** | **Conc. (µM)** | **Inhibiton (%)** | **Ref. CMP** |
|---|---|---|---|---|
| **343** | kappa (h) (KOP) (agonist radioligand) | 5 | 63 | U50488 |
| **343** | 5-HT2A (h) (agonist radioligand) | 5 | 51 | (±)DOI |
| **343** | 5-HT2B (h) (agonist radioligand) | 5 | 70 | (±)DOI |
| **343** | norepinephrine transporter (h) (antagonist radioligand) | 5 | 78 | protriptyline |
| **343** | dopamine transporter (h) (antagonist radioligand) | 5 | 70 | BTCP |
| **343** | COX1 (h) | 5 | 56 | Diclofenac |
| **354** | 5-HT2A (h) (agonist radioligand) | 5 | 73 | (±)DOI |
| **354** | 5-HT2B (h) (agonist radioligand) | 5 | 65 | (±)DOI |
| **354** | norepinephrine transporter (h) (antagonist radioligand) | 5 | 74 | protriptyline |
| **354** | dopamine transporter (h) (antagonist radioligand) | 5 | 64 | BTCP |

**Table 3. Protein families tested for in vitro pharmacology profile.**

| **Family** | ***n*** |
|---|---|
| **GPCR** | 24 |
| **Ion Channel** | 8 |
| **Kinase** | 1 |
| **Lipid Metabolism** | 2 |
| **NHR** | 2 |
| **Neurotransmitter Metabolism** | 2 |
| **PDE** | 2 |
| **Transporter** | 3 |

### Example 3: Evaluation of neuroprotective properties and in vivo pharmacokinetics

To evaluate the *in vitro* efficacy of our newly developed compounds, primary mouse neuronal cultures (PNCs) were employed and glutamate-induced excitotoxicity assays were conducted. Cultures were pre-treated with compounds disclosed herein before glutamate exposure, and neuronal viability was assessed 15 hours post-stimulation with glutamate. Notably, several benzoxazoles according to Formula (I) and the anthranilic acid according to Formula (II) significantly reduced glutamate excitotoxicity ranging from 5% to 50%, confirming their biological activity. The activity of additional compounds might be masked by their observed toxicity at the tested concentrations of 5 µM (see e.g. compounds designated as CMP139, 353, 354, 355, 362, 369, 372, 381 and 457 in Fig. 3A, and 5 and 10-12).

### Example 4: Excitotoxicity assay of benzoxazole and anthranilic acid compounds according to the invention

Biological activity assays conducted on representative benzoxazole compounds according to Formula (I) and anthranilic acid compounds according to Formula (II) revealed up to a 50% reduction in excitotoxicity.

For example, the *in vitro* excitotoxicity profile of benzoxazole CMP312 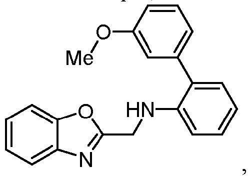 was evaluated resulting in non-toxicity and potent protective effects in the excitotoxicity assay (see Fig. 1C and 3A).

In particular, the ability of benzoxazole CMP312 to inhibit the deterioration of mitochondrial membrane potential (ΔΨm) in response to glutamate-induced excitotoxicity was assessed. The ratio between the ΔΨm-dependent dye tetramethylrhodamine ethyl ester (TMRE) and the mitochondrial dye MitoTracker was quantified for this purpose. Treatment with CMP312 mitigated the glutamate-induced decrease in ΔΨm, thus affirming its protective properties (see Fig. 3B).

### Example 5: In vivo availability

To assess *in vivo* availability of representative compounds from the groups of benzoxazole according to Formula (I), anthranilic acid according to Formula (II) and 5-(5-Isoxazolyl)-2-thiophenesulfonamide according to Formula (III), drug metabolism and pharmacokinetics (DMPK) were evaluated. Compound concentrations in plasma, heart, and brain were measured at several time points after intraperitoneal (i.p.) or intravenous (i.v.) single-dose injections. Especially, compounds from the groups of anthranilic acid according to Formula (II) and 5-(5-Isoxazolyl)-2-thiophenesulfonamide according to Formula (III) showed high levels in plasma and heart, indicating good bioavailability. In particular, the exposure levels reached by compounds in the heart are adequate to warrant testing in models of heart disease at adequate dosing, such as those resulting from TRPM4 gain-of-function mutations.

**Table 4. Summary of in vivo pharmacokinetic properties.**

| | **Route** | **CMP233** | **CMP157** | **CMP354** | **CMP343** |
|---|---|---|---|---|---|
| **IC50** | | 0.15 µM | 0.63 µM | 0.19 µM | 0.09 µM |
| **Bioavailability** | | 95% | 104% | 55% | 57% |
| **Plasma half-life** | IP | 0.9 h | 2.9 h | 4.5 h | 4.3 h |
| **Heart half-life** | IP | 1.1 h | 3.3 h | 4.3 h | 153 h |
| **Brain half-life** | IP | 1.4 h | 3.9 h | 2.6 h | 2.1 h |
| **Dose** | IP | 10 mg kg⁻¹ | 5 mg kg⁻¹ | 10 mg kg⁻¹ | 10 mg kg⁻¹ |
| | | | | | |
| **Plasma half-life** | IV | 0.5 h | 2.8 h | 1.5 h | 0.3 h |
| **Heart half-life** | IV | 1.3 h | 3.5 h | 4.8 h | 2.1 h |
| **Brain half-life** | IV | 0.3 h | 4.2 h | 1.6 h | 0.2 h |
| **Dose** | IV | 3 mg kg⁻¹ | 2.5 mg kg⁻¹ | 3 mg kg⁻¹ | 3 mg kg⁻¹ |

### Example 6: TRPM4 binding site

*In silico* comparison revealed that binding of small molecule typically occurs in cavities above the Ca²⁺-binding site, as seen in collared flycatcher (cf) TRPM8 ⁶¹ (SEQ ID NO: 9), or involving the transmembrane helices S3-S4 and the pore forming domain, with helix 3 being involved in Ca²⁺-binding, as observed in human TRPV1 ⁶²⁻⁶⁴ (SEQ ID NO: 10), rabbit TRPV5⁶⁵ (SEQ ID NO: 11), human TRPA1 ⁶⁶ (SEQ ID NO: 12) and zebrafish (dr) TRPM5 ⁶⁷ (SEQ ID NO: 5). Notably, the interaction of N'-(3,4-dimethoxybenzylidene)-2-(naphthalen-1-yl)acetohydrazide (NDNA) with drTRPM5 (SEQ ID NO: 5), due to its structural similarity to TRPM4 (SEQ ID NO: 1) and comparable size to compounds disclosed herein, offered valuable insights. A structural comparison, aligning NDNA-bound drTRPM5 and hsTRPM4, suggested the conservation of a hydrophobic binding cavity in hsTRPM4. The cavity appeared more spacious towards the cytosolic interface, attributed to the substitution of E835 and N792 in drTRPM5 (SEQ ID NO: 13) by S863 and S924 in hsTRPM4 (SEQ ID NO: 7) respectively. Furthermore, the cytosolic interface of the cavity is lined by the TRP domain, crucial for channel gating, where N990 in drTRPM5 (SEQ ID NO: 5) is substituted by R1064 in human TRPM4 (SEQ ID NO: 1) and S1060 in mouse TRPM4 (SEQ ID NO: 8) (Fig. 4A, 7).

The structure-based method DoGSite was used to predict binding pockets and employed the support vector machine-based DoGSiteScorer to assess the drugability of these predicted sites. In the Ca²⁺-bound hsTRPM4 ³⁰, a binding pocket with a drugability score of 0.73, hydrophobicity of 0.51 and a volume of 329 Å³ was predicted, adequately sized to accommodate the compounds disclosed herein. Similarly, in the ligand-free (apo) drTRPMS, a binding pocket with a drugability score of 0.73, hydrophobicity of 0.49 and a volume of 350 Å³ was predicted (Fig. 4A, 7). In drTRPMS, Ca²⁺ binding triggers the relative movement between the S4-S5 linker and the TRP domain, facilitating the repositioning of the pore forming SS-S6 helices and channel opening. Consequently, the binding site of NDNA is well-positioned to interfere with this movement, and NDNA-binding stabilizes Ca²⁺-bound drTRPM5 in an apo-like closed state ⁶⁷. In TRPM4, Ca²⁺binding is thought to precede voltage-dependent opening ³¹ with gating mechanisms believed to operate similarly ⁶⁸. *In silico* docking of compounds disclosed herein to TRPM4 shows a binding configuration (Fig. 7) that is comparable to the binding of NDNA to TRPM5.

To validate this hypothesis, hsTRPM4 mutants with alanine substitutions (L907A, SEQ ID NO: 6; and S924A, SEQ ID NO: 6) were genetically engineered with the aim to disrupt this proposed compound binding site. L907A, corresponding to I836 in drTRPM5 (SEQ ID NO: 5), was hypothesized to reduce the hydrophobic interactions within the cavity. Alongside, S924A, corresponding to E853 in drTRPM5 (SEQ ID NO: 5), was hypothesized to affect compound binding in a manner similar to NDNA in drTRPM5 (Fig. 4A). Initially, the basic properties of the hsTRPM4 mutants (SEQ ID NOs: 6 and 7) were assessed using whole-cell patch clamp electrophysiology. Both mutants, L907A and S924A (SEQ ID NOs: 6 and 7), exhibited the typical time course of activation for WT TRPM4 (SEQ ID NO: 1), as was described before ⁶⁹. Notably, both mutants appeared as gain-of-function variants, exhibiting an increase in current density at the initial peak and at the plateau during steady state (Fig. 4B, 8A). These findings correlated with a 3.5-fold increase in Ca²⁺ sensitivity of the mutants (Fig. 4C, 8B).

Subsequently, the inhibition of hsTRPM4 wildtype (SEQ ID NO: 1) and L907A and S924A mutants (SEQ ID NOs: 6 and 7) was compared by selected compounds according to the invention, i.e. benzoxazoles 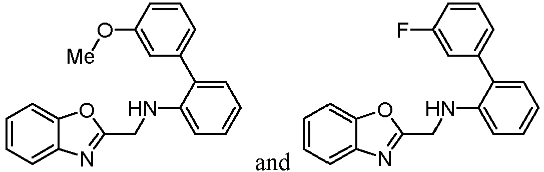 as well as anthranilic acid 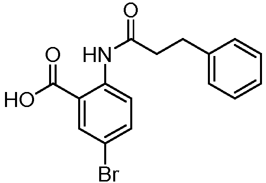 (i.e. compounds also designated herein as CMP312, CMP343, and CMP233, respectively) alongside the known TRPM4 antagonist CBA ⁵⁷. All tested compounds demonstrated significantly reduced current inhibition in at least one hsTRPM4 mutant variant (SEQ ID NOs: 6 and 7), whereas inhibition by CBA remained unaffected. Moreover, in the L907A/S924A double mutant (SEQ ID NO: 14) the affinity for the benzoxazole of the invention CMP312 and the anthranilic acid of the invention CMP233 is completely abolished (Fig. 4D, 9).

Since it has been reported that CBA inhibits human TRPM4 efficiently but not mouse TRPM4⁷⁰, the species specificity of compounds disclosed herein was evaluated. The activity of compounds disclosed herein and CBA on both human and mouse TRPM4 (SEQ ID NOs: 1 and 8) using the inside-out patch clamp configuration. Benzoxazole compounds according to Formula (I) 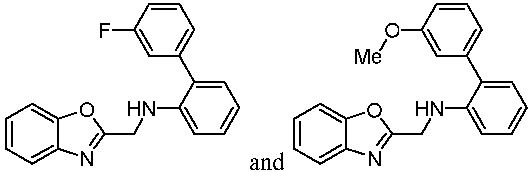 (also designated as CMP343 and CMP312) showed reduced activity on mouse TRPM4 (SEQ ID NO: 8) compared to human TRPM4 (SEQ ID NO: 1), with currents of human TRPM4 almost completely blocked. In contrast, the anthranilic acid compound according to Formula (II) 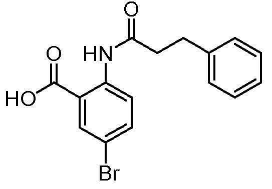 (also designated as CMP233) was highly effective in both species, unlike the anthranilic acid ortholog antagonist CBA, which exhibited no activity on mouse TRPM4 (Fig. 4E, 10).

Upon investigating potential binding pockets of the novel TRPM4-binding compounds disclosed herein through comparative analyses within the TRP channel family, the present inventors identified two primary binding sites: one located directly above the Ca²⁺-binding site enclosed by the transmembrane helices S1-4 ^{30,78}, and another opposite of the S1 domain ⁶⁷, situated near the TRP and pore-forming domains. Given the narrow structure-activity-relationship (SAR) of benzoxazole compounds according to Formula (I) disclosed herein and its compliance with the space limitation of this site, the present inventors explored alanine substitutions of hsTRPM4 L907 and S924 (SEQ ID NOs: 6and 7), corresponding to drTRPM5 I836A (SEQ ID NO: 15) and E853A (SEQ ID NO: 13), respectively ⁶⁷. In drTRPM5 (SEQ ID NO: 5), these modifications preserved channel functionality but reduced sensitivity to NDNA. Notably, both TRPM4 mutants exhibited an increase in current density, coupled with a 3-fold rise in Ca²⁺ sensitivity. This underscores the close proximity of the compound binding site to TRPM4's Ca²⁺-binding site and potential interaction between these sites. Crucially, the affinity of these variants for both benzoxazoles according to Formula (I) and anthanilic acids according to Formula (II) disclosed herein, but not for CBA, was significantly reduced. This indicated that L907 and S924 are involved in the binding compounds from both series.

In drTRPM5 (SEQ ID NO: 5), substitution of L833A (SEQ ID NO: 16) and W869A (SEQ ID NO: 17), corresponding to L904 and W940 in hsTRPM4 (SEQ ID NO: 1), led to agonistic activity of NDNA ⁶⁷. Similarly, in TRPV1 (SEQ ID NO: 10), the vanilloid agonists resinifera-toxin ⁷⁹, and in TRPA1 (SEQ ID NO: 12), the agonist GNE55 1 ⁶⁶, bind to a pocket homologous to the NDNA-binding site. These findings suggest that this common binding site indeed possesses allosteric properties, wherein minor changes can lead to a switch from antagonistic to agonistic activity and vice versa.

Together, these results strongly support the interaction of compounds described herein with the proposed binding site located in a small pocket within the transmembrane region between Ca²⁺-binding and pore-forming domain and offer insights into species-specific activities of these compounds.

### Example 7: Comparison of compound activities between mouse and human TRPM4

Binding of compounds disclosed herein to mouse and human TRPM4 was investigated. The present inventors observed that binding of representative benzoxazoles according to Formula (I), i.e. CMP343 and 312 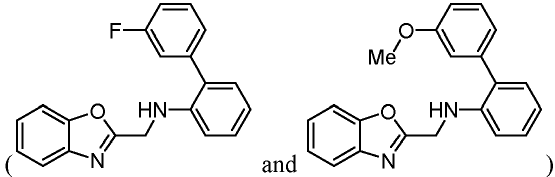 is significantly less effective on mouse TRPM4 (SEQ ID NO: 8), whereas anthranilic acid CMP233 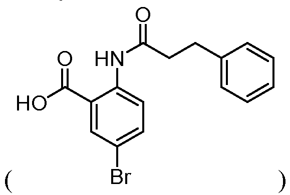 is active on both species. In contrast known TRPM4 antagonist CBA only affects human TRPM4 (SEQ ID NO: 1) as reported previously⁷⁰. Accordingly, compounds disclosed herein can advantageously be applied to a broader spectrum of subjects, in particular human and mouse subjects, compared to known TRPM4 antagonist CBA.

The proposed binding site exhibits only three different amino acids between human and mouse TRPM4 (SEQ ID NOs: 1 and 8, respectively). While V904, corresponding to L900 in mouse TRPM4, leads to minor changes at the top of the hydrophobic pocket, its influence may not be significant. However, S868 and R1064, corresponding to T859 and S1060 in mouse TRPM4 (SEQ ID NO: 8), respectively, are positioned at the cytosolic interface of the proposed binding pocket (Fig. 7). Without wishing to be bound by theory, these residues may be involved in binding or modulating accessibility to benzoxazole compounds according to Formula (I) disclosed herein. Additionally, these residues may impede the binding of CBA, particularly with chlorine at position 4 of the anthranilic acid.

### Methods

### EXPERIMENTAL MODELS AND STUDY PARTICIPANT DETAILS

### Cell culture

HEK293 cells parental cells, TRPM4- and TRPM5-expressing HEK293 cells (Anaxon AG, Bern, Switzerland) were cultured in DMEM (Thermo Fisher Scientific, 10566016) supplemented with 10% FCS (PAN, P30-3306) at 37 °C and 5% CO₂. Cells were split every 3 to 4 days at a confluence of approximately 70%. Primary mouse neuronal cultures were prepared following the methods outlined previously ²⁶. Briefly, cortices or hippocampi from E15.5 embryos were isolate and subjected to trypsin digestion for 6 minutes at 37 °C. The digestion process was stopped using DMEM/F-12 (Thermo Fisher Scientific, 10565018) supplemented with 10% fetal calf serum (FCS), followed by tissue washing with HBSS (Thermo Fisher Scientific, 14170112). Subsequently, cells were dissociated in PNGM (Lonza, CC-4461). Dissociated cells were seeded at a density of 60,000 cells per cm² onto poly-L-lysine-coated (5 µM; Sigma-Aldrich, A-003-M) well plates or glass cover slips and cultured at 37 °C and 5% CO₂ for a minimum of 14 days before further experimentation.

### METHOD DETAILS

### Small molecule library

The compounds identified by the present inventors to be effective in inhibiting the function of a TRPM4 ion channel are commercially available from a compound library from Evotec SE, consisting of 256,286 compounds with high chemical diversity of compounds. Additionally, previously unknown derivatives of benzoxazoles according to Formula (I) as disclosed herein (also referred to as "Series 1 compounds") have been synthesized by methods known in the art.

### FLIPR assay

HEK293 parental cells and HEK293 cells stably expressing human TRPM4 or TRPM5 (Anaxon AG, Bern, Switzerland) were seeded at 10,000 cells per well in 50 µl growth medium supplemented with 9% FCS in 384-well plates. Next day, cells were washed once in FLIPR Membrane Potential Assay Kit (Molecular Devices). Subsequently, the buffer was aspirated to a residual volume of 25 µl before addition of 25 µl FLIPR membrane potential (FMP) blue dye (Molecular Devices, R8034). Cells were incubated for 60 min at 37 °C before placement into the FLIPR Tetra High-Throughput Cellular Screening System (Molecular Devices). For the first FLIPR-read, 5 µl of the test compounds, the positive control TRPM4 antagonist 9-phenanthrol or FAA, vehicle control DMSO 0.25% or activation control ionomycin were added and fluorescence was measured for 140 seconds. Finally, 5 µl ionomycin in a final concentration of 1 µM was added to induce intracellular Ca²⁺ rise that activates TRPM4 and the second FLIPR-read was obtained for an additional 140 seconds.

### QPatch assay

QPatchHTX, an automated electrophysiology platform, was used in single hole mode. One day before use human TRPM4- or TRPM5-expressing HEK293 cells were seeded in a T75 flask at a density of 53k cells per cm² and at passages < 23. For QPatch assays, cells were harvested and re-suspended in SFM (Gibco, 11686029). The extracellular solution was (in mM): CaCl₂ (7), MgCl₂ (2), KCl (5), HEPES (10), NaCl (70), NMDG (70); pH 7.4 with NaOH. The intracellular solution was (in mM): HEPES (10), NMDG (100), NaCl (50), EGTA (20), MgCl₂ (1), CaCl₂ (21); pH 7.2 with HCl. After break-in, channels were consistently open and current through the channels could be observed at a voltage ramp from -100 mV to +100 mV over 500 ms. This protocol was repeated every 7 seconds. After saline and vehicle application, the channel was desensitized and residual current was used as baseline. FFA was used as reference compound and applied cumulatively with each concentration incubating at a minimum of 20 voltage protocol runs (140 seconds) with the current after addition of FFA being calculated as full block. Compounds were prepared from 20 mM stocks in DMSO and first measured at 0.3, 3 and 30 µM concentrations to establish 3-point IC50 curves. If compounds were potent, concentrations were adjusted to 0.003, 0.03, 0.3, 3 and 30 µM to obtain precise IC50 values. Each compound was measured at least 3 time independently.

### Excitotoxicity assays

Excitotoxicity assays were performed using primary mouse neuronal cultures between 14 and 17 days *in vitro* (DIV). Cell viability was measured using RealTime-Glo MT Cell Viability Assay (Promega, catalog no. G9711) according to the manufacture protocol. Compounds were co-administered with RealTime-Glo reagents, and luciferase measurements were conducted using the Tecan-Spark M10 microplate reader with environmental control set to 37 °C and 5% CO₂. Neuronal cultures were stimulated with either glutamate or vehicle five hours after compound application and monitored for an additional 20 hours. To adjust for inter-well variability, arbitrary light units were normalized to a steady signal prior to glutamate addition for each well. Toxicity was calculated relative to the minimal cell viability at 15 hours post glutamate stimulation and cell viability of the PBS vehicle control.

### TMRE assay

Primary mouse neuronal cultures were grown on µ-Dish 35 mm Quad dishes (ibidi, 80416) and were treated after 15-17 d.i.v. with either vehicle or CMP312 for 30 minutes. Subsequently 50 µM glutamate was applied for in total 2 hours. Then, 20 nM TMRE dye (Abcam, ab113852), 500 nM MitoTracker dye (ThermoFisher, M7512), and 1 µM Hoechst 33342 were added after 1.5 hours glutamate stimulation for 30 minutes. Afterwards cells were washed two times in prewarmed medium and were imaged immediately in preconditioned medium using a confocal microscope (Zeiss, LSM700) with 20-fold magnification and controlled atmosphere conditions (37 °C, 5% CO₂). Imaging time was less than 10 minutes per condition. We quantified the total MFI of the MitoTracker and TMRE dyes per cell. At least 50 cells per condition were quantified and the mean ratios of TMRE to Mitotracker per independent experiments were reported.

### In vivo pharmacokinetics

Three mice per group and time point were injected intravenously or intraperitoneally using the following concentrations per bodyweight: CMP354, CMP343 and CMP233 i.v. 3 mg kg⁻¹, i.p. 10 mg kg⁻¹; CMP157 i.v. 2.5 mg kg⁻¹, i.p. 5 mg kg⁻¹. Animals were sacrificed after 5, 15, 60, 120, 240, 420 or 1440 minutes post injection and compound concentrations were determined by mass spectrometry in plasma, heart and brain. Half-life and bioavailability were calculated by noncompartmental analysis using the R package PK ⁸¹ (v1.3-6).

### Hepatocyte clearance

Cryopreserved hepatocytes were exposed to test Compounds at a concentration of 1 µM for 120 minutes. Samples were collected at various time points, and percent remaining of test compound was determined using mass spectrometry (LC-MS/MS) to calculate hepatic clearance rate (L/h/kg).

### Microsome clearance

Human or mouse liver microsomes were incubated with the test compound at a concentration of 1 µM for 45 min in the presence of cofactor (NADPH in excess). Samples were collected at various time points, and % remaining of test compound was determined using mass spectrometry (LC-MS/MS) to calculate hepatic clearance rate (L/h/kg).

### CYP inhibition assay

The half-maximal inhibitory concentration (IC50) for cytochrome P450 (CYP) enzymes was determined using an in vitro enzyme inhibition assay. Recombinant CYP enzymes or human liver microsomes from BD Gentest, in 0.1 M phosphate buffer pH 7.4, containing native CYP enzymes were incubated with the test compound at various concentrations (1, 3, 10 and 30 µM) and containing 0.5% DMSO and 0.1% BSA final concentrations. 1 mM of NADPH cofactor was added and the reaction was started by a rapid increase of temperature until 37 °C. The IC50 value was determined as the concentration of the test compound required to inhibit 50% of the enzyme activity by measuring metabolite formation and was calculated using appropriate dose-response curve fitting methods.

### Caco-2 permeability assay

Caco-2 permeability was evaluated using a cell-based assay with Caco-2 cells cultured on permeable membrane inserts. The test compound was added at 10 µM to the apical (donor) compartment, and samples were collected from the basolateral (receiver) compartment at 120 min. The test compound was also added at 10 µM to the basolateral (donor) compartment, and samples were collected from the apical (receiver) compartment at 120 min. The amount of the compound in both compartments was measured using mass spectrometry (LC-MS/MS). The permeability coefficient (Papp, nm/s) apical to basolateral and basolateral to apical, and an efflux ratio were calculated.

### PAMPA

Parallel Artificial Membrane Permeability Assay (PAMPA) was performed using artificial lipid membrane layers to assess passive permeability of the test compound. Test compounds were diluted to 200 µM in system buffer at pH 5.0, 6.2 and 7.4. The solutions were filtered and 150 µL transferred to a High Sensitivity 96 well UV plate. This was analysed by UV as the reference plate. A 200 µL sample of the 200 µM solution was transferred to the 'donor' plate of the PAMPA pIon sandwich plate system. 200 µL of acceptor sink buffer was transferred to the 'acceptor' plate which had been previously treated with GIT-O lipid solution across the well filter. Donor and acceptor plates were sandwiched and kept in a humid environment at room temperature for 16 hours. On completion of the incubation the donor and acceptor plates were separated. 150 µL of solution was transferred from each of the donor and acceptor PAMPA sandwich plates into High Sensitivity 96 well UV plates for analysis by UV. at different time points. The concentration of the compound in the acceptor compartment was determined using mass spectrometry. The permeability coefficient (Papp) was calculated based on the rate of appearance of the compound in the acceptor compartment (nm/s).

### In vitro pharmacology

For *in vitro* pharmacological profiling and determination of off target effects compound were screened at a 5 µM in duplicate for their potential to bind to a broad panel of receptors, enzymes, and ion channels on a commercially available platform (Safetyscreen 44 Panel; Eurofins Cerep, Le Bois l'Evêque, B.P. 30001, 86 600 Celle l'Evescault, France), Study ID FR095-0009609).

### Molecular biology

Human *TRPM4* (SEQ ID NO: 1; UniProtKB Accession number: Q8TD43-1) and mouse *Trpm4* (SEQ ID NO: 8; UniProtKB Accession number: Q7TN37-1) were amplified via PCR and inserted into the lentiviral vector pFUGW (Addgene #14883), which harbors a custom multiple cloning site and a C-terminal eGFP tag, using the restriction enzymes NheI and Pfl32II. Additionally, human TRPM4 was inserted into the pcDNA3.1 plasmid using the same restriction enzymes. TRPM4 mutants were generated by site-directed mutagenesis using the Q5 Site-Directed Mutagenesis Kit (Promega, E0554S).

### Electrophysiology

Patch clamp recordings were conducted using an EPC10 patch clamp amplifier and Patchmaster software (HEKA, Germany). An Ag/AgCl wire served as the reference electrode in all experiments. Patch pipettes, pulled from borosilicate capillary tubes using a DMZ universal puller, exhibited final resistances ranging from 2 to 4 MQ. Liquid junction potential was compensated before achieving gigaseal formation. All experiments were conducted at room temperature. Whole-cell currents were recorded in transiently transfected HEK293 cells expressing either wildtype human TRPM4 (SEQ ID NO: 1), TRPM4-L907A (SEQ ID NO: 6), or TRPM4-S924A (SEQ ID NO: 7). Recordings involved a 400 ms increasing voltage ramp from -100 mV to +100 mV, initiated from a holding potential of 0 mV. The interval between each sweep was 2 seconds, with data sampled at 5 kHz. The extracellular solution composition was as follows (in mM): NaCl (156), HEPES (10), glucose (10), CaCl₂ (1.5), and MgCl₂ (1), pH 7.4. To identify TRPM4 currents, extracellular Na⁺ was replaced with equimolar NMDG⁺. Inside-out currents were recorded from membrane patches excised from transiently transfected HEK-293T cells expressing either wildtype human TRPM4 (SEQ ID NO: 1), TRPM4-L907A (SEQ ID NO: 6), TRPM4-S924A (SEQ ID NO: 7), or wildtype mouse TRPM4 (SEQ ID NO: 8). Similar to whole-cell recordings, a 400 ms increasing voltage ramp from -100 mV to +100 mV was applied from a holding potential of 0 mV. The interval between sweeps was 2 seconds, and data were sampled at 5 kHz. The pipette solution comprised (in mM): NaCl (156), HEPES (10), glucose (10), CaCl₂ (1.5), and MgCl₂ (1), pH 7.4, while the intracellular solution contained (in mM): NaCl (20), NaAsp (120), HEPES (10), and MgCl₂ (1), pH 7.2. Transiently transfected HEK-293T cells were sealed, and membrane patches were excised using the intracellular solution, supplemented with 10 mM EGTA. TRPM4 currents were activated by 500 µM CaCl₂ and deactivated by 10 mM EGTA, both added to the intracellular solution. Ca²⁺ activation curves were fitted using a likelihood maximization algorithm to estimate the parameters for the sigmoidal fitting curves y-maximum, slope and midpoint as part of the R package sicegar (V0.2.4).

### Multidimensional scaling

Compounds were canonicalized using the Open Babel library ⁸² and R package ChemmineR (v3.17). Extended molecular fingerprints of the compounds and Tanimoto similarity matrices were calculated using R package RCDK (v3.8.1).

### Modelling and compound docking

The human TRPM4 structure (SEQ ID NO: 1) in a calcium-bound state (PDB ID:6bqv) and the zebrafish TRPM5 (SEQ ID NO: 5) in a calcium and NDNA-bound state (PDB ID:7mbv) were aligned using helices S3, S4 and S5' as reference. Alignments and visualization were performed using the PyMol software. Identification of druggable cavities and docking of compounds were carried out using DoGSiteScorer ⁸³ and JAMDA ⁸⁴, respectively, as provided by the Proteins*Plus* modeling tools ⁸⁵.

### QUANTIFICATION AND STATISTICAL ANALYSIS

The statistical analyses applied are detailed in the respective legends to the figures and include the number of biological replicates (N) and the statistical test used. Significant results are indicated by **P* < 0.05, ***P* < 0.01, ****P* < 0.001.

### REFERENCES

1. Pivovarov, A.S., Calahorro, F., and Walker, R.J. (2019). Na+/K+-pump and neurotransmitter membrane receptors. Invert. Neurosci. 19, 1. https://doi.org/10.1007/s10158-018-0221-7.
2. Berridge, M.J., Bootman, M.D., and Roderick, H.L. (2003). Calcium signalling: dynamics, homeostasis and remodelling. Nat. Rev. Mol. Cell Biol. 4, 517-529. https://doi.org/10.1038/nrm1155.
3. Madden, D.R. (2002). The structure and function of glutamate receptor ion channels. Nat. Rev. Neurosci. 3, 91-101. https://doi.org/10.1038/nm725.
4. Coyle, J.T., and Puttfarcken, P. (1993). Oxidative Stress, Glutamate, and Neurodegenerative Disorders. Science 262, 689-695. https://doi.org/10.1126/science.7901908.
5. Yuan, J., and Ofengeim, D. (2023). A guide to cell death pathways. Nat. Rev. Mol. Cell Biol. https://doi.org/10.1038/s41580-023-00689-6.
6. Bano, D., and Ankarcrona, M. (2018). Beyond the critical point: An overview of excitotoxicity, calcium overload and the downstream consequences. Neurosci. Lett. 663, 79-85. https://doi.org/10.1016/j.neulet.2017.08.048.
7. Arundine, M., and Tymianski, M. (2003). Molecular mechanisms of calcium-dependent neurodegeneration in excitotoxicity. Cell Calcium 34, 325-337. https://doi.org/10.1016/S0143-4160(03)00141-6.
8. Hardingham, G.E., Fukunaga, Y., and Bading, H. (2002). Extrasynaptic NMDARs oppose synaptic NMDARs by triggering CREB shut-off and cell death pathways. Nat. Neurosci. 5, 405-414. https://doi.org/10.1038/nn835.
9. Randall, R., and Thayer, S. (1992). Glutamate-induced calcium transient triggers delayed calcium overload and neurotoxicity in rat hippocampal neurons. J. Neurosci. 12, 1882-1895. https://doi.org/10.1523/JNEUROSCI.12-05-01882.1992.
10. Mahmoud, S., Gharagozloo, M., Simard, C., and Gris, D. (2019). Astrocytes Maintain Glutamate Homeostasis in the CNS by Controlling the Balance between Glutamate Uptake and Release. Cells 8, 184. https://doi.org/10.3390/cells8020184.
11. Barger, S.W., and Basile, A.S. (2001). Activation of microglia by secreted amyloid precursor protein evokes release of glutamate by cystine exchange and attenuates synaptic function. J. Neurochem. 76, 846-854. https://doi.org/10.1046/j.1471-4159.2001.00075.x.
12. Barger, S.W., Goodwin, M.E., Porter, M.M., and Beggs, M.L. (2007). Glutamate release from activated microglia requires the oxidative burst and lipid peroxidation. J. Neurochem. 101, 1205-1213. https://doi.org/10.1111/j.1471-4159.2007.04487.x.
13. Takeuchi, H., Jin, S., Wang, J., Zhang, G., Kawanokuchi, J., Kuno, R., Sonobe, Y., Mizuno, T., and Suzumura, A. (2006). Tumor Necrosis Factor-α Induces Neurotoxicity via Glutamate Release from Hemichannels of Activated Microglia in an Autocrine Manner. J. Biol. Chem. 281, 21362-21368. https://doi.org/10.1074/jbc.M600504200.
14. Birkner, K., Wasser, B., Ruck, T., Thalman, C., Luchtman, D., Pape, K., Schmaul, S., Bitar, L., Krämer-Albers, E.-M., Stroh, A., et al. (2020). β1-Integrin- and KV1.3 channel-dependent signaling stimulates glutamate release from Th17 cells. J. Clin. Invest. 130, 715-732. https://doi.org/10.1172/JCI126381.
15. Binvignat, O., and Olloquequi, J. (2020). Excitotoxicity as a Target Against Neurodegenerative Processes. Curr. Pharm. Des. 26, 1251-1262. https://doi.org/10.2174/1381612826666200113162641.
16. Olloquequi, J., Cornejo-Córdova, E., Verdaguer, E., Soriano, F.X., Binvignat, O., Auladell, C., and Camins, A. (2018). Excitotoxicity in the pathogenesis of neurological and psychiatric disorders: Therapeutic implications. J. Psychopharmacol. (Oxf.) 32, 265-275. https://doi.org/10/gczcw4.
17. Witte, M.E., Schumacher, A.-M., Mahler, C.F., Bewersdorf, J.P., Lehmitz, J., Scheiter, A., Sánchez, P., Williams, P.R., Griesbeck, O., Naumann, R., et al. (2019). Calcium Influx through Plasma-Membrane Nanoruptures Drives Axon Degeneration in a Model of Multiple Sclerosis. Neuron 101, 615-624.e5. https://doi.org/10.1016/j.neuron.2018.12.023.
18. Lees, K.R., Asplund, K., Carolei, A., Davis, S.M., Diener, H.-C., Kaste, M., Orgogozo, J.-M., and Whitehead, J. (2000). Glycine antagonist (gavestinel) in neuroprotection (GAIN International) in patients with acute stroke: a randomised controlled trial. The Lancet 355, 1949-1954. https://doi.org/10.1016/50140-6736(00)02326-6.
19. Lipton, S.A. (2006). Paradigm shift in neuroprotection by NMDA receptor blockade: Memantine and beyond. Nat. Rev. Drug Discov. 5, 160-170. https://doi.org/10.1038/nrd1958.
20. Moore, T.J., Alami, A., Alexander, G.C., and Mattison, D.R. (2022). Safety and effectiveness of NMDA receptor antagonists for depression: A multidisciplinary review. Pharma-cother. J. Hum. Pharmacol. Drug Ther. 72, 567-579. https://doi.org/10.1002/phar.2707.
21. Muir, K. (2006). Glutamate-based therapeutic approaches: clinical trials with NMDA antagonists. Curr. Opin. Pharmacol. 6, 53-60. https://doi.org/10.1016/j.coph.2005.12.002.
22. Hanson, J.E., Yuan, H., Perszyk, R.E., Banke, T.G., Xing, H., Tsai, M.-C., Menniti, F.S., and Traynelis, S.F. (2024). Therapeutic potential of N-methyl-D-aspartate receptor modulators in psychiatry. Neuropsychopharmacology 49, 51-66. https://doi.org/10.1038/s41386-023-01614-3.
23. Perszyk, R.E., Swanger, S.A., Shelley, C., Khatri, A., Fernandez-Cuervo, G., Epplin, M.P., Zhang, J., Le, P., Bülow, P., Garnier-Amblard, E., et al. (2020). Biased modulators of NMDA receptors control channel opening and ion selectivity. Nat. Chem. Biol. 16, 188-196. https://doi.org/10.1038/s41589-019-0449-5.
24. Yan, J., Bengtson, C.P., Buchthal, B., Hagenston, A.M., and Bading, H. (2020). Coupling of NMDA receptors and TRPM4 guides discovery of unconventional neuroprotectants. 9. https://doi.org/10.1126/science.aay3302.
25. Aarts, M., Liu, Y., Liu, L., Besshoh, S., Arundine, M., Gurd, J.W., Wang, Y.-T., Salter, M.W., and Tymianski, M. (2002). Treatment of Ischemic Brain Damage by Perturbing NMDA Receptor- PSD-95 Protein Interactions. Science 298, 846-850. https://doi.org/10.1126/science.1072873.
26. Woo, M.S., Ufer, F., Rothammer, N., Di Liberto, G., Binkle, L., Haferkamp, U., Sonner, J.K., Engler, J.B., Hornig, S., Bauer, S., et al. (2021). Neuronal metabotropic glutamate receptor 8 protects against neurodegeneration in CNS inflammation. J. Exp. Med. 218, e20201290. https://doi.org/10.1084/jem.20201290.
27. Harris, J.R., and Boekema, E.J. eds. (2018). Membrane Protein Complexes: Structure and Function (Springer Singapore) https://doi.org/10.1007/978-981-10-7757-9.
28. Huang, Y., Fliegert, R., Guse, A.H., Lü, W., and Du, J. (2020). A structural overview of the ion channels of the TRPM family. Cell Calcium 85, 102111. https://doi.org/10.1016/j.ceca.2019.102111.
29. Vennekens, R., Mesuere, M., and Philippaert, K. (2018). TRPM5 in the battle against diabetes and obesity. Acta Physiol. 222, e12949. https://doi.org/10.1111/apha.12949.
30. Autzen, H.E., Myasnikov, A.G., Campbell, M.G., Asarnow, D., Julius, D., and Cheng, Y. (2018). Structure of the human TRPM4 ion channel in a lipid nanodisc. Science 359, 228-232. https://doi.org/10/gcn44s.
31. Nilius, B., Prenen, J., Droogmans, G., Voets, T., Vennekens, R., Freichel, M., Wissen-bach, U., and Flockerzi, V. (2003). Voltage Dependence of the Ca2+-activated Cation Channel TRPM4. J. Biol. Chem. 278, 30813-30820. https://doi.org/10.1074/jbc.M305127200.
32. Bousova, K., Jirku, M., Bumba, L., Bednarova, L., Sulc, M., Franek, M., Vyklicky, L., Vondrasek, J., and Teisinger, J. (2015). PIP2 and PIP3 interact with N-terminus region of TRPM4 channel. Biophys. Chem. 205, 24-32. https://doi.org/10.1016/j.bpc.2015.06.004.
33. Guo, J., She, J., Zeng, W., Chen, Q., Bai, X., and Jiang, Y. (2017). Structures of the calcium-activated, non-selective cation channel TRPM4. Nature 552, 205. https://doi.org/10/gcm4zb.
34. Stokum, J.A., Kwon, M.S., Woo, S.K., Tsymbalyuk, O., Vennekens, R., Gerzanich, V., and Simard, J.M. (2018). SUR1-TRPM4 and AQP4 form a heteromultimeric complex that amplifies ion/water osmotic coupling and drives astrocyte swelling. Glia 66, 108-125. https://doi.org/10.1002/glia.23231.
35. Stokum, J.A., Shim, B., Negoita, S., Tsymbalyuk, N., Tsymbalyuk, O., Ivanova, S., Keledjian, K., Bryan, J., Blaustein, M.P., Jha, R.M., et al. (2023). Cation flux through SUR1-TRPM4 and NCX1 in astrocyte endfeet induces water influx through AQP4 and brain swelling after ischemic stroke. Sci. Signal. 16, eadd6364. https://doi.org/10.1126/scisignal.add6364.
36. Becerra, A., Echeverría, C., Varela, D., Sarmiento, D., Armisén, R., Nuñez-Villena, F., Montecinos, M., and Simon, F. (2011). Transient receptor potential melastatin 4 inhibition prevents lipopolysaccharide-induced endothelial cell death. Cardiovasc. Res. 91, 677-684. https://doi.org/10.1093/cvr/cvr135.
37. Fearey, B.C., Binkle, L., Mensching, D., Schulze, C., Lohr, C., Friese, M.A., Oertner, T.G., and Gee, C.E. (2022). A glibenclamide-sensitive TRPM4-mediated component of CA1 excitatory postsynaptic potentials appears in experimental autoimmune encephalomyelitis. Sci. Rep. 12, 6000. https://doi.org/10.1038/s41598-022-09875-6.
38. Woo, S.K., Tsymbalyuk, N., Tsymbalyuk, O., Ivanova, S., Gerzanich, V., and Simard, J.M. (2020). SUR1-TRPM4 channels, not KATP, mediate brain swelling following cerebral ischemia. Neurosci. Lett. 718, 134729. https://doi.org/10.1016/j.neulet.2019.134729.
39. Schattling, B., Steinbach, K., Thies, E., Kruse, M., Menigoz, A., Ufer, F., Flockerzi, V., Brück, W., Pongs, O., Vennekens, R., et al. (2012). TRPM4 cation channel mediates axonal and neuronal degeneration in experimental autoimmune encephalomyelitis and multiple sclerosis. Nat. Med. 18, 1805-1811. https://doi.org/10/f4fmk4.
40. Chen, B., Gao, Y., Wei, S., Low, S.W., Ng, G., Yu, D., Tu, T.M., Soong, T.W., Nilius, B., and Liao, P. (2019). TRPM4-specific blocking antibody attenuates reperfusion injury in a rat model of stroke. Pflüg. Arch. - Eur. J. Physiol. 471, 1455-1466. https://doi.org/10.1007/s00424-019-02326-8.
41. Loh, K.P., Ng, G., Yu, C.Y., Fhu, C.K., Yu, D., Vennekens, R., Nilius, B., Soong, T.W., and Liao, P. (2014). TRPM4 inhibition promotes angiogenesis after ischemic stroke. Pflüg. Arch. - Eur. J. Physiol. 466, 563-576. https://doi.org/10.1007/s00424-013-1347-4.
42. Gerzanich, V., Woo, S.K., Vennekens, R., Tsymbalyuk, O., Ivanova, S., Ivanov, A., Geng, Z., Chen, Z., Nilius, B., Flockerzi, V., et al. (2009). De novo expression of Trpm4 initiates secondary hemorrhage in spinal cord injury. Nat. Med. 15, 185-191. https://doi.org/10.1038/nm.1899.
43. Simard, J.M., Kahle, K.T., and Gerzanich, V. (2010). Molecular mechanisms of microvascular failure in central nervous system injury-synergistic roles of NKCC1 and SUR1/TRPM4: A review. J. Neurosurg. 113, 622-629. https://doi.org/10.3171/2009.11.JNS081052.
44. Wang, H., Xu, Z., Lee, B.H., Vu, S., Hu, L., Lee, M., Bu, D., Cao, X., Hwang, S., Yang, Y., et al. (2019). Gain-of-Function Mutations in TRPM4 Activation Gate Cause Progressive Symmetric Erythrokeratodermia. J. Invest. Dermatol. 139, 1089-1097. https://doi.org/10.1016/j .jid.2018.10.044.
45. Vandewiele, F., Pironet, A., Jacobs, G., Kecskés, M., Wegener, J., Kerselaers, S., Hen-drikx, L., Verelst, J., Philippaert, K., Oosterlinck, W., et al. (2022). TRPM4 inhibition by meclofenamate suppresses Ca2+-dependent triggered arrhythmias. Eur. Heart J. 43, 4195-4207. https://doi.org/10.1093/eurheartj/ehac354.
46. Guo, Y., Yu, Z.-Y., Wu, J., Gong, H., Kesteven, S., Iismaa, S.E., Chan, A.Y., Holman, S., Pinto, S., Pironet, A., et al. (2021). The Ca2+-activated cation channel TRPM4 is a positive regulator of pressure overload-induced cardiac hypertrophy. eLife 10, e66582. https://doi.org/10.75 54/eLife.665 82.
47. Kecskés, M., Jacobs, G., Kerselaers, S., Syam, N., Menigoz, A., Vangheluwe, P., Freichel, M., Flockerzi, V., Voets, T., and Vennekens, R. (2015). The Ca2+-activated cation channel TRPM4 is a negative regulator of angiotensin II-induced cardiac hypertrophy. Basic Res. Cardiol. 110, 43. https://doi.org/10.1007/s00395-015-0501-x.
48. Kruse, M., Schulze-Bahr, E., Corfield, V., Beckmann, A., Stallmeyer, B., Kurtbay, G., Ohmert, I., Schulze-Bahr, E., Brink, P., and Pongs, O. (2009). Impaired endocytosis of the ion channel TRPM4 is associated with human progressive familial heart block type I. J. Clin. Invest. 119, 2737-2744. https://doi.org/10.1172/JCI38292.
49. Mathar, I., Vennekens, R., Meissner, M., Kees, F., Van der Mieren, G., Camacho Londoño, J.E., Uhl, S., Voets, T., Hummel, B., van den Bergh, A., et al. (2010). Increased catecholamine secretion contributes to hypertension in TRPM4-deficient mice. J. Clin. Invest. 120, 3267-3279. https://doi.org/10.1172/JCI41348.
50. Piao, H., Takahashi, K., Yamaguchi, Y., Wang, C., Liu, K., and Naruse, K. (2015). Transient Receptor Potential Melastatin-4 Is Involved in Hypoxia-Reoxygenation Injury in the Cardiomyocytes. PLOS ONE 10, e0121703. https://doi.org/10.1371/journal.pone.0121703.
51. Barbet, G., Demion, M., Moura, I.C., Serafini, N., Léger, T., Vrtovsnik, F., Monteiro, R.C., Guinamard, R., Kinet, J.-P., and Launay, P. (2008). The calcium-activated nonselective cation channel TRPM4 is essential for the migration but not the maturation of dendritic cells. Nat. Immunol. 9, 1148-1156. https://doi.org/10.1038/ni.1648.
52. Launay, P., Cheng, H., Srivatsan, S., Penner, R., Fleig, A., and Kinet, J.-P. (2004). TRPM4 Regulates Calcium Oscillations After T Cell Activation. Science 306, 1374-1377. https://doi.org/10.1126/science.1098845.
53. Shimizu, T., Owsianik, G., Freichel, M., Flockerzi, V., Nilius, B., and Vennekens, R. (2009). TRPM4 regulates migration of mast cells in mice. Cell Calcium 45, 226-232. https://doi.org/10.1016/j.ceca.2008.10.005.
54. Vennekens, R., Olausson, J., Meissner, M., Bloch, W., Mathar, I., Philipp, S.E., Schmitz, F., Weissgerber, P., Nilius, B., Flockerzi, V., et al. (2007). Increased IgE-dependent mast cell activation and anaphylactic responses in mice lacking the calcium-activated nonselective cation channel TRPM4. Nat. Immunol. 8, 312-320. https://doi.org/10.1038/ni1441.
55. Demion, M., Bois, P., Launay, P., and Guinamard, R. (2007). TRPM4, a Ca2+-activated nonselective cation channel in mouse sino-atrial node cells. Cardiovasc. Res. 73, 531-538. https://doi.org/10.1016/j.cardiores.2006.11.023.
56. Woo, S.K., Kwon, M.S., Ivanov, A., Gerzanich, V., and Simard, J.M. (2013). The Sulfonylurea Receptor 1 (Sur1)-Transient Receptor Potential Melastatin 4 (Trpm4) Channel. J. Biol. Chem. 288, 3655-3667. https://doi.org/10/f4mqhs.
57. Ozhathil, L.C., Delalande, C., Bianchi, B., Nemeth, G., Kappel, S., Thomet, U., Ross-Kaschitza, D., Simonin, C., Rubin, M., Gertsch, J., et al. (2018). Identification of potent and selective small molecule inhibitors of the cation channel TRPM4: Potent TRPM4 inhibitors. Br. J. Pharmacol. 175, 2504-2519. https://doi.org/10.1111/bph.14220.
58. Liu, Y., Mathes, C., Friis, S., and Finley, M. (2009). QPatch: The Missing Link Between HTS and Ion Channel Drug Discovery. Comb. Chem. High Throughput Screen. 12, 78-95. https://doi.org/10.2174/138620709787047948.
59. Guinamard, R., Simard, C., and Del Negro, C. (2013). Flufenamic acid as an ion channel modulator. Pharmacol. Ther. 138, 272-284. https://doi.org/10.1016/j.pharmthera.2013.01.012.
60. Bowes, J., Brown, A.J., Hamon, J., Jarolimek, W., Sridhar, A., Waldron, G., and Whitebread, S. (2012). Reducing safety-related drug attrition: the use of in vitro pharmacological profiling. Nat. Rev. Drug Discov. 11, 909-922. https://doi.org/10.1038/nrd3845.
61. Yin, Y., Le, S.C., Hsu, A.L., Borgnia, M.J., Yang, H., and Lee, S.-Y. (2019). Structural basis of cooling agent and lipid sensing by the cold-activated TRPM8 channel. Science, eaav9334. https://doi.org/10/gfvm99.
62. Neuberger, A., Oda, M., Nikolaev, Y.A., Nadezhdin, K.D., Gracheva, E.O., Bagriantsev, S.N., and Sobolevsky, A.I. (2023). Human TRPV1 structure and inhibition by the analgesic SB-366791. Nat. Commun. 14, 2451. https://doi.org/10.1038/s41467-023-38162-9.
63. Yang, F., Xiao, X., Cheng, W., Yang, W., Yu, P., Song, Z., Yarov-Yarovoy, V., and Zheng, J. (2015). Structural mechanism underlying capsaicin binding and activation of the TRPV1 ion channel. Nat. Chem. Biol. 11, 518-524. https://doi.org/10.1038/nchembio.1835.
64. Nadezhdin, K.D., Neuberger, A., Nikolaev, Y.A., Murphy, L.A., Gracheva, E.O., Bagriantsev, S.N., and Sobolevsky, A.I. (2021). Extracellular cap domain is an essential component of the TRPV1 gating mechanism. Nat. Commun. 12, 2154. https://doi.org/10.1038/s41467-021-22507-3.
65. Hughes, T.E.T., Lodowski, D.T., Huynh, K.W., Yazici, A., Del Rosario, J., Kapoor, A., Basak, S., Samanta, A., Han, X., Chakrapani, S., et al. (2018). Structural basis of TRPV5 channel inhibition by econazole revealed by cryo-EM. Nat. Struct. Mol. Biol. 25, 53-60. https://doi.org/10.1038/s41594-017-0009-1.
66. Liu, C., Reese, R., Vu, S., Rougé, L., Shields, S.D., Kakiuchi-Kiyota, S., Chen, H., Johnson, K., Shi, Y.P., Chemov-Rogan, T., et al. (2021). A Non-covalent Ligand Reveals Biased Agonism of the TRPA1 Ion Channel. Neuron 109, 273-284.e4. https://doi.org/10.1016/j.neuron.2020.10.014.
67. Ruan, Z., Haley, E., Orozco, I.J., Sabat, M., Myers, R., Roth, R., Du, J., and Lü, W. (2021). Structures of the TRPM5 channel elucidate mechanisms of activation and inhibition. Nat. Struct. Mol. Biol. 28, 604-613. https://doi.org/10.1038/s41594-021-00607-4.
68. Duan, J., Li, Z., Li, J., Santa-Cruz, A., Sanchez-Martinez, S., Zhang, J., and Clapham, D.E. (2018). Structure of full-length human TRPM4. Proc. Natl. Acad. Sci. 115, 2377-2382. https://doi.org/10/gc8gvz.
69. Ullrich, N.D., Voets, T., Prenen, J., Vennekens, R., Talavera, K., Droogmans, G., and Nilius, B. (2005). Comparison of functional properties of the Ca2+-activated cation channels TRPM4 and TRPM5 from mice. Cell Calcium 37, 267-278. https://doi.org/10.1016/j.ceca.2004.11.001.
70. Arullampalam, P., Preti, B., Ross-Kaschitza, D., Lochner, M., Rougier, J.-S., and Abriel, H. (2021). Species-Specific Effects of Cation Channel TRPM4 Small-Molecule Inhibitors. Front. Pharmacol. 12, 712354. https://doi.org/10.3389/fphar.2021.712354.
71. EU Pharmaceutical reform: Steering innovation to address unmet medical needs (2024).
72. Jönsson, L. (2022). The personal economic burden of dementia in Europe. Lancet Reg. Health - Eur. 20, 100472. https://doi.org/10.1016/j.lanepe.2022.100472.
73. Tay, L.X., Ong, S.C., Tay, L.J., Ng, T., and Parumasivam, T. (2024). Economic Burden of Alzheimer's Disease: A Systematic Review. Value Health Reg. Issues 40, 1-12. https://doi.org/10.1016/j.vhri.2023.09.008.
74. Mathur, S., Gawas, C., Ahmad, I.Z., Wani, M., and Tabassum, H. (2023). Neurodegenerative disorders: Assessing the impact of natural vs drug-induced treatment options. AGING Med. 6, 82-97. https://doi.org/10.1002/agm2.12243.
75. Ureshino, Erustes, Bassani, Wachilewski, Guarache, Nascimento, Costa, Smaili, and Pereira (2019). The Interplay between Ca2+ Signaling Pathways and Neurodegeneration. Int. J. Mol. Sci. 20, 6004. https://doi.org/10.3390/ijms20236004.
76. Pchitskaya, E., Popugaeva, E., and Bezprozvanny, I. (2018). Calcium signaling and molecular mechanisms underlying neurodegenerative diseases. Cell Calcium 70, 87-94. https://doi.org/10.1016/j.ceca.2017.06.008.
77. Soloveva, V., Larocque, J., and Mckillip, E. (2006). When Robots Are Good: Fully Automated Thermo LAS Robotic Assay System with Dual FLIPRTETRA and TAP SelecT Robotic Cell Culture System. JALA J. Assoc. Lab. Autom. 11, 145-156. https://doi.org/10.1016/jjala.2006.02.006.
78. Nadezhdin, K.D., Talyzina, I.A., Parthasarathy, A., Neuberger, A., Zhang, D.X., and Sobolevsky, A.I. (2023). Structure of human TRPV4 in complex with GTPase RhoA. Nat. Commun. 14, 3733. https://doi.org/10.1038/s41467-023-39346-z.
79. Cao, E., Liao, M., Cheng, Y., and Julius, D. (2013). TRPV1 structures in distinct conformations reveal activation mechanisms. Nature 504, 113-118. https://doi.org/10.1038/nature12823.
80. Chen, B., Ng, G., Gao, Y., Low, S.W., Sandanaraj, E., Ramasamy, B., Sekar, S., Bhakoo, K., Soong, T.W., Nilius, B., et al. (2018). Non-Invasive Multimodality Imaging Directly Shows TRPM4 Inhibition Ameliorates Stroke Reperfusion Injury. Transl. Stroke Res. 10, 91-103. https://doi.org/10.1007/s12975-018-0621-3.
81. Jaki, T., and Wolfsegger, M.J. (2011). Estimation of pharmacokinetic parameters with the R package PK. Pharm. Stat. 10, 284-288. https://doi.org/10.1002/pst.449.
82. O'Boyle, N.M., Banck, M., James, C.A., Morley, C., Vandermeersch, T., and Hutchison, G.R. (2011). Open Babel: An open chemical toolbox. J. Cheminformatics 3, 33. https://doi.org/10.1186/1758-2946-3-33.
83. Volkamer, A., Kuhn, D., Grombacher, T., Rippmann, F., and Rarey, M. (2012). Combining Global and Local Measures for Structure-Based Druggability Predictions. J. Chem. Inf. Model. 52, 360-372. https://doi.org/10.1021/ci200454v.
84. Flachsenberg, F., Meyder, A., Sommer, K., Penner, P., and Rarey, M. (2020). A Consistent Scheme for Gradient-Based Optimization of Protein - Ligand Poses. J. Chem. Inf. Model. 60, 6502-6522. https://doi.org/10.1021/acs.jcim.0c01095.
85. Schöning-Stierand, K., Diedrich, K., Ehrt, C., Flachsenberg, F., Graef, J., Sieg, J., Penner, P., Poppinga, M., Ungethüm, A., and Rarey, M. (2022). Proteins Plus: a comprehensive collection of web-based molecular modeling tools. Nucleic Acids Res. 50, W611-W615. https://doi.org/10.1093/nar/gkac305.

## Claims

1. Benzoxazole according to 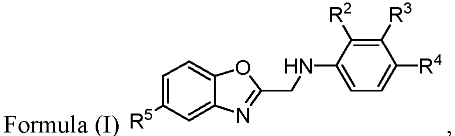 wherein
R2 is selected from H, F, Cl, Br, I, substituted or unsubstituted aryl or heteroaryl groups, substituted or unsubstituted C₁ to C₁₀ alkyl, in particular CF₃, amine groups NR⁶R⁷ with R6 and R7 independently selected from H, and C₁ to C₄ alkyl, in particular, Me, Et, Pr, iPr, hydroxy or alkoxy groups OR⁸ with R8 being selected from H, and C₁ to C₄ alkyl, in particular, Me, Et, Pr, iPr, Bu and 5 to 10 membered non-annulated or annulated aryl or heteroaryl,
preferably R2 is
phenyl according to 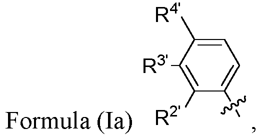 wherein
R2' is selected from H, F, Cl, Br, I, substituted or unsubstituted C₁ to C₁₀ alkyl, in particular CF₃, amine groups NR^{6'}R^{7'} with R6' and R7' independently selected from H, and C₁ to C₄ alkyl, in particular, Me, Et, Pr, iPr, Bu,; and
R3' and R4' are independently selected from H and amine groups NR^{6'}R^{7'} with R6' and R7' independently selected from H, and C₁ to C₄ alkyl, in particular, Me, Et, Pr, iPr, Bu,
preferably R2' is selected from H, F and NH₂, R3' is selected from H, NH₂, NHMe and NHiPr, and R4' is selected from H and NHMe;
or
heteroaryl according to 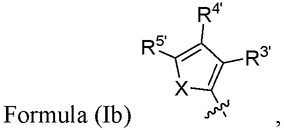
wherein
X is selected from N, O and S;
R3', R4' and R5' are independently selected from H, F, Cl, Br, I and substituted or unsubstituted C₁ to C₁₀ alkyl, in particular Me and Et,
preferably X is S and R3' and R5' are independently selected from H and Me and R4' is H;
R3 and R4 are H;
R5 is selected from H, F, Cl, Br, I and substituted or unsubstituted C₁ to C₁₀ alkyl, preferably R5 is H.

2. Benzoxazole according to claim 1, selected from the group consisting of 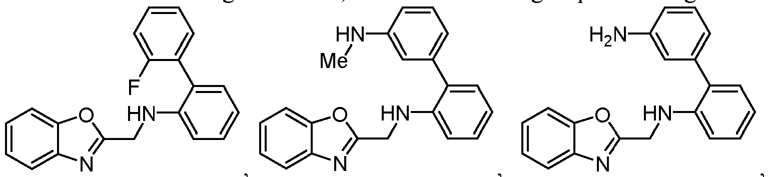 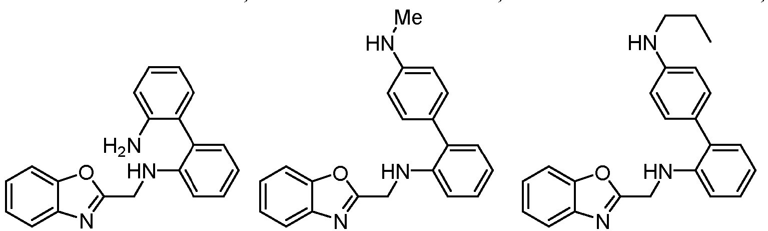 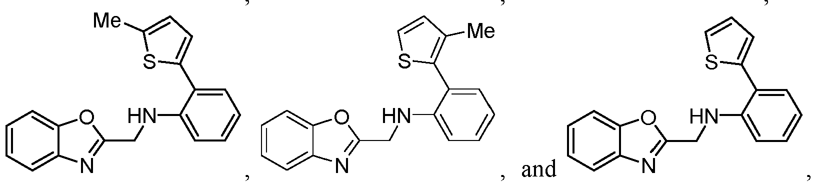 preferably the benzoxazole according to Formula (I) is

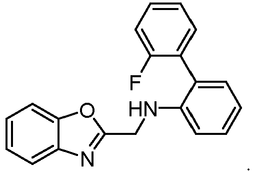

3. Compound which is effective in inhibiting the function of a TRPM4 ion channel for use in a method of treating or preventing a TRMP4-associated disorder in a subject, wherein said compound is selected from
a) benzoxazole according to 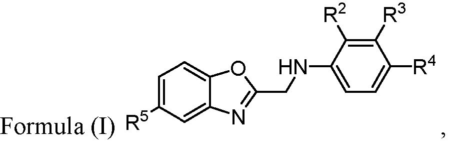 wherein
R2 is selected from H, F, Cl, Br, I, substituted or unsubstituted C₁ to C₁₀ alkyl, in particular CF₃, amine groups NR⁶R⁷ with R6 and R7 independently selected from H, and C₁ to C₄ alkyl, in particular, Me, Et, Pr, iPr, hydroxy or alkoxy groups OR⁸ with R8 being selected from H, and C₁ to C₄ alkyl, in particular, Me, Et, Pr, iPr, Bu and 5 to 10 membered non-annulated or annulated aryl or heteroaryl,
preferably R2 is
phenyl according to 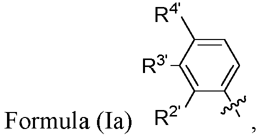 wherein
R2', R3' and R4' are independently selected from H, F, Cl, Br, I, substituted or unsubstituted C₁ to C₁₀ alkyl, in particular CF₃, amine groups NR⁶ R⁷ with R6' and R7' independently selected from H, and C₁ to C₄ alkyl, in particular, Me, Et, Pr, iPr, Bu and hydroxy or alkoxy groups OR^{8'} with R8' being selected from H, and C₁ to C₄ alkyl, in particular, Me, Et, Pr, iPr, Bu;
or
heteroaryl according to 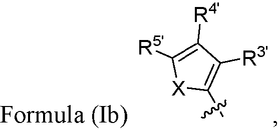
wherein
X is selected from N, O and S;
R3', R4' and R5' are independently selected from H, F, Cl, Br, I and substituted or unsubstituted C₁ to C₁₀ alkyl, in particular Me and Et;
R3 and R4 are independently selected from H, and unsubstituted or halosubstituted Me or Et;
R5 is selected from H, F, Cl, Br, I and substituted or unsubstituted C₁ to C₁₀ alkyl;
b) anthranilic acid according to 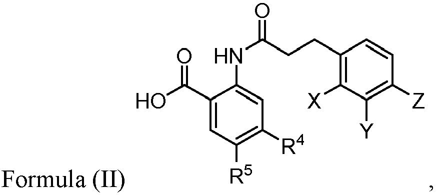 wherein
R4 and Z are independently selected from H and substituted or unsubstituted C₁ to C₁₀ alkyl, in particular CF₃;
R5, X and Y are independently selected from H, F, Cl, Br, I and halosubstituted or unsubstituted C₁ to C₁₀ alkyl, in particular CF₃.
c) 5-(5-Isoxazolyl)-2-thiophenesulfonamide according to 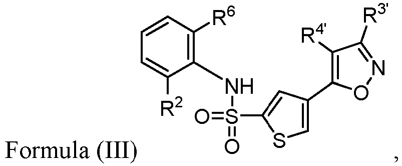 wherein
R2 is selected from Hand C₁ to C₁₀ alkyloxy groups, in particular methoxy, ethoxy and propyloxy;
R6 is selected from H, F, Cl, Br, I, halosubstituted or unsubstituted C₁ to C₁₀ alkyl, in particular CF₃, and 5 to 10 membered non-annulated or annulated aryl or heteroaryl, in particular phenyl;
R3' and R4' are independently selected from H and substituted or unsubstituted C₁ to C₁₀ alkyl, in particular Me, Et, Pr, iPr and CF₃;
d) benzpyrimidine according to 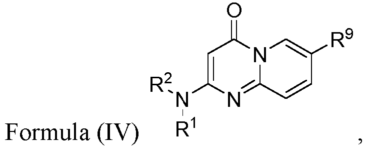 wherein
R1 and R2 are independently selected from Hand C₁ to C₈ alkyl and cycloalkyl, with R1 and R2 optionally forming a fused ring system;
R9 is selected from H, F, Cl, Br, I, halosubstituted or unsubstituted C₁ to C₁₀ alkyl, in particular CF₃;
and/or
e) thiadiazole according to 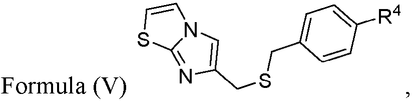 wherein
R4 is selected from H, F, Cl, Br, I, halosubstituted or unsubstituted C₁ to C₁₀ alkyl, in particular CF₃
or a pharmaceutically acceptable salt, solvate, tautomer or ester thereof.

4. Compound for use according to claim 3, wherein the compound is a benzoxazole according to 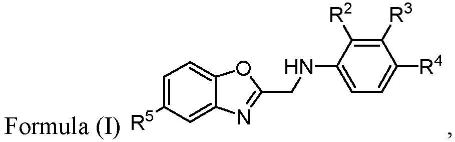 wherein R2 is
phenyl according to 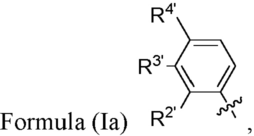 wherein
R2' is selected from H, F, NH₂ and OMe,
R3' is selected from H, F, NH₂, NHMe, NHPr,
R4' is selected from H, NHMe or
heteroaryl according to 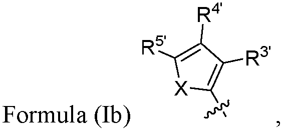 wherein
X is S; and
R3' and R5' are independently selected from H and Me,
R4' is H,
and
R3, R4 and R5 each are H.

5. Compound for use according to any one of claims 3 or 4, wherein the compound is a benzoxazole according to Formula (I) selected from the group consisting of 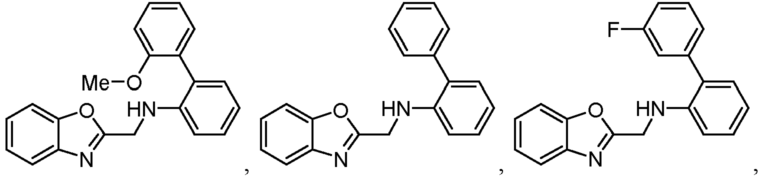 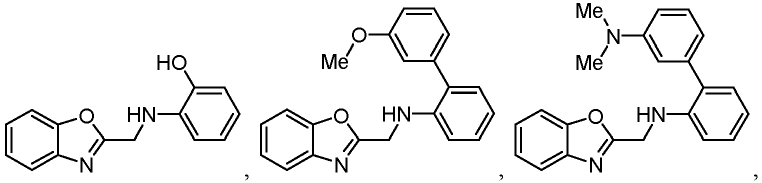 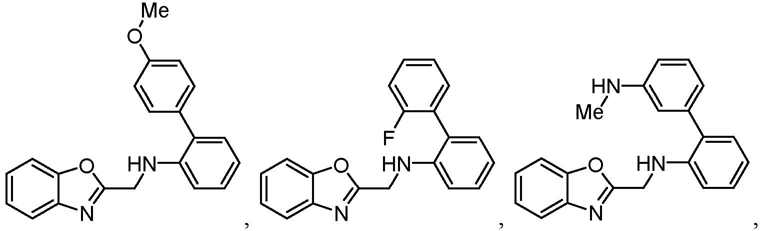 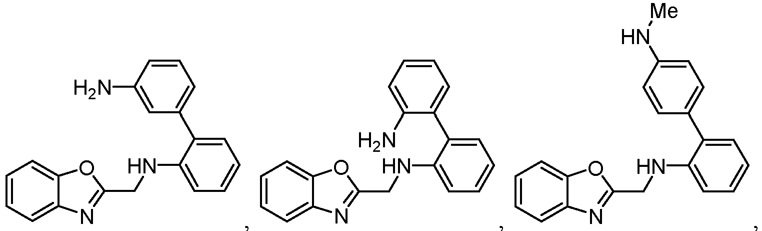 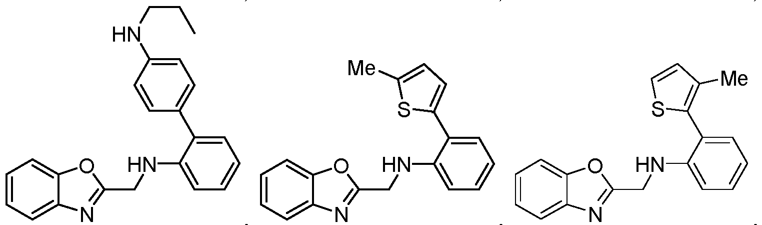 and 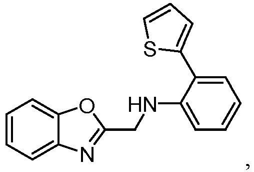 preferably the benzoxazole according to Formula (I) is selected from the group consisting of 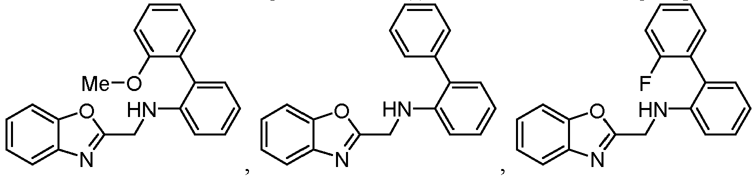 and 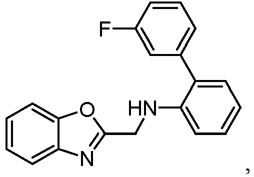 most preferably the benzoxazole according to Formula (I) is 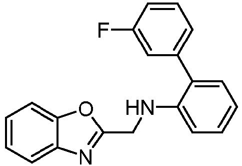 or

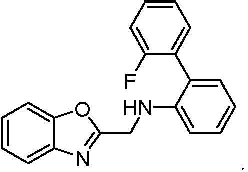

6. Compound for use according to claim 3, wherein the compound is an anthranilic acid according to Formula (II) 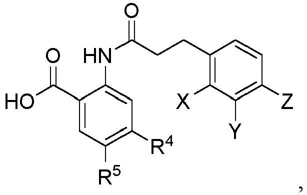 wherein
X and Y each are independently selected from Hand Cl,
R4 and Z each are H,
R5 is selected from Cl, Br and CF₃,
preferably, the compound is an anthranilic acid according to Formula (II) selected from the group consisting of 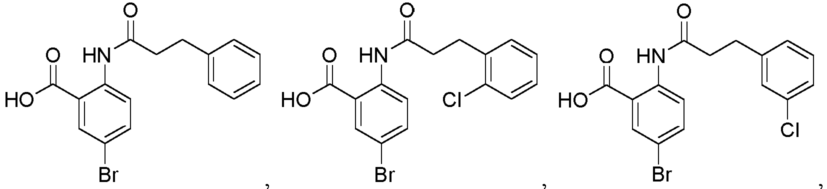 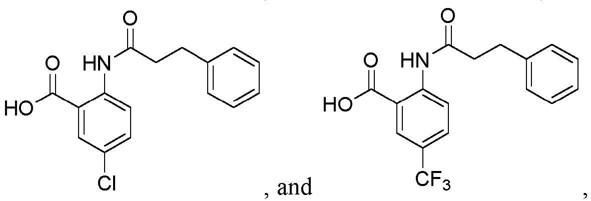
more preferably the anthranilic acid according to Formula (II) is

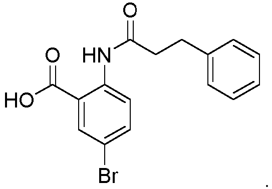

7. Compound for use according to claim 3, wherein the compound is a 5-(5-Isoxazolyl)-2-thiophenesulfonamide according to 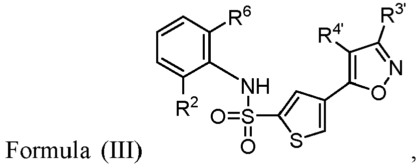 wherein R3' and R4' both are Me, preferably, the compound is a 5-(5-Isoxazolyl)-2-thiophenesulfonamide according to Formula (III) selected from the group consisting of 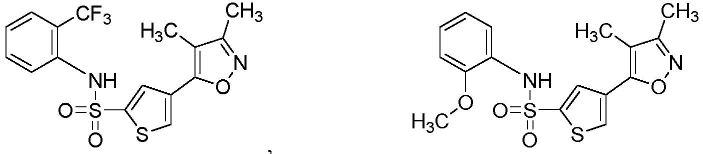 and 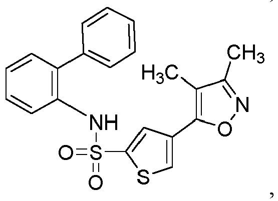 more preferably the 5-(5-Isoxazolyl)-2-thiophenesulfonamide according to Formula (III) is

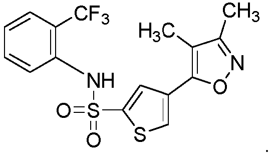

8. Compound for use according to claim 3, wherein the compound is a benzpyrimidine according to 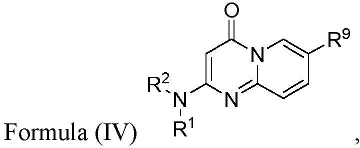 wherein
R1 is H and R2 is cycloheptyl and R9 is selected from H, F, Cl, Br, I and CF₃,
preferably the benzpyrimidine according to Formula (IV) is

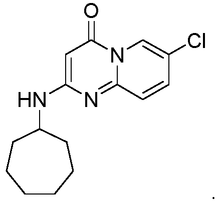

9. Compound for use according to claim 3, wherein the compound is a thiadiazole according to 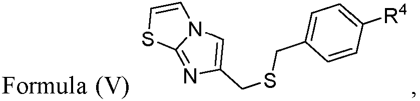 wherein R4 is H or Cl, preferably the thiadiazole according to Formula (V) is selected from the group consisting of 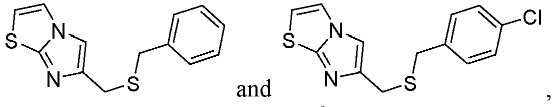 more preferably the thiadiazole according to Formula (V) is

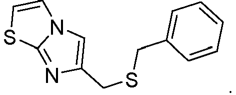

10. Compound for use according to any one of claims 3 to 9, wherein the TRMP4-associated disorder is associated with glutamate excitotoxicity.

11. Compound for use according to any one of claims 3 to 10, wherein the TRMP4-associated disorder is selected from neurodegenerative diseases, genodermatosis, cardiac diseases, and immune-mediated diseases, preferably the TRMP4-associated disorder is a neurodegenerative disease, more preferably the TRMP4-associated disorder is a neurodegenerative disease selected from stroke, Alzheimer's disease, Parkinson's disease, amyotrophic lateral sclerosis, and neurodegenerative disease that is associated with inflammation, in particular multiple sclerosis, preferably, wherein the TRMP4-associated disorder is multiple sclerosis.

12. Compound for use according to any one of claims 3 to 11, wherein the subject is a mammal, preferably human or mouse, more preferably human.

13. Compound for use according to any of claims 3 to 12, wherein the compound is to be administered in an amount from 1.0 µg/kg body weight to 10,000 µg/kg body weight.

14. Compound for use according to any of claims 3 to 13, wherein the compound binds to TRMP4 at least via amino acids L907 and S924 of SEQ ID NO: 1.

15. Pharmaceutical composition comprising the compound according to any one of claims 1 to 14, wherein said composition further comprises at least one excipient, diluent, carrier and/or at least one additional pharmaceutically active compound which is useful in treating or preventing a TRMP4-associated disorder in a subject.
